(19) Europäisches Patentamt
European Patent Office
Office européen des brevets

(11) Publication number: **0 366 066 B1**

# (12) EUROPEAN PATENT SPECIFICATION

(45) Date of publication of patent specification: **01.03.95**

(51) Int. Cl.[6]: **C07C 257/18**, C07C 323/32, C07D 233/14, C07D 233/16, A61K 31/155, A61K 31/415

(21) Application number: **89119720.4**

(22) Date of filing: **24.10.89**

## (54) Compounds and formulations for use in methods for the treatment and prophylaxis of pneumocystis cariniipneumonia and other diseases.

(30) Priority: **25.10.88 US 262535**
**25.10.88 US 262324**
**06.04.89 US 334590**
**06.04.89 US 334730**

(43) Date of publication of application:
**02.05.90 Bulletin 90/18**

(45) Publication of the grant of the patent:
**01.03.95 Bulletin 95/09**

(84) Designated Contracting States:
**AT BE CH DE ES FR GB GR IT LI LU NL SE**

(56) References cited:
**DE-C- 862 602**
**US-A- 2 277 861**

**THE AMERICAN JOURNAL OF TROPICAL MEDICINE AND HYGIENE, vol. 32, no. 2, March 1983, pages 231-257; L.H. SCHMIDT: "Appraisals of compounds of diverse chemical classes for capacities to cure infections with sporozoites of plasmodium cynomolgi"**

(73) Proprietor: **THE UNIVERSITY OF NORTH CAROLINA AT CHAPEL HILL**
**300 Bynum Hall, Campus Box 4100**
**Chapel Hill, North Carolina 27599-4100 (US)**

Proprietor: **THE UNITED STATES GOVERNMENT as represented by THE SECRETARY OF THE ARMY**
**Intellectual Property Law Division**
**Office of the Judge Advocate General,DA**
**Suite 700, 901 N. Stuart St.**
**Arlington, Virginia 22203-1837 (US)**

(72) Inventor: **Tidwell, Richard R.**
**101 Forest Ridge Drive**
**Chapel Hill, North Carolina (US)**
Inventor: **Geratz, J. Dieter**
**713 Kenmore Road**
**Chapel Hill, North Carolina (US)**
Inventor: **Ohemeng, Kwasi A.**
**112 Overlook Drive**
**Clinton, New Jersey (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

EXPERIMENTAL PARASITOLOGY, vol. 52, no. 3, December 1981, pages 404-413, Academic Press: E.A. STECK et al.: "Leishmania donovani, Plasmodium berghei, Trypanosoma rhodesiense: Antiprotozoal Effects of Some Amidine Types"

LIEBIGS ANNALEN DER CHEMIE, vol. 10, 1982, pages 1836-1869, O. DANN et al.: "Synthesen biskationischer, trypanocider 1-Benzofuran-Verbindungen"

CHEMICAL ABSTRACTS, vol. 78, no. 11, March 1973, page 447, column 1, abstract no. 72150g, Columbus, Ohio, US; & DE-A-2 122 208

JOURNAL OF THE CHEMICAL SOCIETY, 1942, pages 103-116, GB; J.N. ASHLEY et al.: "A Chemotherapeutic Comparison of the Trypanocidal Action of Some Aromatic Diamidines"

ANNALS OF TROPICAL MEDICINE AND PARASITOLOGY, vol. 69, no. 3, September 1975, pages 311-328, Liverpool University Press; W. PETERS et al.: "The chemotherapy of rodent malaria, XXIII"

Inventor: **Hall, James Edwin**
**2440 Springview Trail**
**Chapel Hill, North Carolina (US)**
Inventor: **Kyle, Dennis E.**
**9415 Curran Road**
**Silver Spring, Maryland (US)**
Inventor: **Grogl, Max**
**3404 Tan Terra Circle**
**Brookville, Maryland (US)**

(74) Representative: **Dost, Wolfgang, Dr.rer.nat., Dipl.-Chem. et al**
**Patent- und Rechtsanwälte**
**Bardehle . Pagenberg . Dost . Altenburg . Frohwitter . Geissler & Partner**
**Postfach 86 06 20**
**D-81633 München (DE)**

## Description

This application relates, in general, to compounds for use in methods for treating Pneumocystis carinii pneumonia and in particular to new compounds which are pharmaceutically active against Pneumocystis carinii, to pharmaceutical formulations containing such compounds, to compounds for use in methods for the treatment of, and prophylaxis against, Pneumocystis carinii pneumonia, and to compounds for use in methods for treating leishmaniasis, giardiasis, and malaria.

## BACKGROUND OF THE INVENTION

### General

Pentamidine, in the form of its hydrochloride salt, was first discovered by Ewins et al., as shown in U.S. Patent No. 2,277,861, and water-soluble salts were subsequently developed as shown by U.S. Patent No. 2,410,796 to Newberry et al, which is directed to such water soluble salts, particularly the hydroxy-ethane sulfonic acid and the hydroxy-propane sulfonic acid salts of pentamidine. The former compound is generally referred to as pentamidine isethionate.

Steck et al. (Experimental Parasitology, 1981, **52(3)**, 404 to 413) investigated diamidines and cyclic congeners for antiprotozoal effects in standard animal models. None of the compounds was found to exhibit appreciable antimalaria or antileishmanial activity. The compound 2,5-bis(4-guanylphenyl)furan dihydrochloride was identified as having antitrypanosomal activity in the same range as pentamidine.

In Liebigs Annalen der Chemie, 1982, **10**, 1836 - 1869, Dann et al. describe syntheses of biscationic, trypanocidal 1-benzofuran compounds with certain substituents, i.a. amidino groups.

### Pneumocystis Carinii Pneumonia

Pentamidine isethionate is presently marketed by LyphoMed, Inc. under the trademark Pentam, for intravenous and intramuscular injection, and is indicated for the treatment of pneumonia due to Pneumocystis carinii, the latter ailment typically being referred as "PCP". The importance of pentamidine isethionate has dramatically escalated recently due to the marked increase of patients suffering from PCP. The increase in the afflicted patient population is an unfortunate consequence of the increasing presence of the Acquired Immunodeficiency Syndrome ("AIDS"). It is now estimated that approximately 70 percent of AIDS patients contract PCP. Because of the high incidence of PCP in AIDS patients, pentamidine isethionate has found utility not only in the treatment of PCP, but also for prophylaxis, in preventing or delaying the initial onset or recurrence of PCP, especially in AIDS patients.

However, an unfortunate side effect of pentamidine isethionate is its toxicity. Some fatalities have been attributed to severe hypotension, hypoglycemia, and cardiac arrhythmias in patients treated with pentamidine isethionate, through both intramuscular and intravenous routes. Because of the concern over the toxicity of pentamidine isethionate, a severe need has arisen for a replacement for pentamidine isethionate which can avoid or minimize the undesirable side effects associated with the use of pentamidine.

### Giardia

Giardia lamblia is the most frequently identified enteric parasite in the United States. Pediatr.Clin. North AM, Jun. 1988, 35(3), 565-77. It is purportedly the most common pathogenic enteric protozoan and is an important cause of gastro-intestinal disease throughout the world. It is an especially critical problem in third-world countries and presents a particularly difficult problem when it infects children.

In one study of infections in malnourished Jamaican children, in those instances of infection where an etiological agent was identified, Giardia lamblia was the most common enteric pathogen. J.Trop. Med. Hyg., 91(4), 173-80, Aug. 1988. In another study of households located in the Nile Delta region of Egypt, involving 724 children, only one child remained giardia-negative during the study. Am. J. Epidemiol., 127(6), 1272-81, Jun. 1988.

Unfortunately, although Giardia lamblia is such a ubiquitous pathogenic protozoan, inhabiting the upper portion of the small intestines, causing both acute and chronic diarrhea and malabsorption, the presently used therapeutic agents are less than satisfactory. In fact, the therapeutic agents currently of choice were developed principally for treatment of other infections and later found to be efficacious against Giardia lamblia. Typically, giardiasis is treated with metronidazole, tinidazole, quinacrine, or furazolidone, but such treatment is typically associated with undesirable side effects and is not always successful.

Although pentamidine has been known for decades, it has not heretofore been known to have utility in the treatment of giardiasis.

It goes without saying that in view of the magnitude of Giardia lamblia infection throughout the world, and the lack of a satisfactory agent for the treatment thereof, an urgent need exists for a more effective anti-Giardia agent having good therapeutic properties.

## Leishmania

Leishmania are well known intra-cellular protozoan parasites which may give rise to serious infections in man. The organisms are transmitted by the "bite" of an infected sandfly, and invade the reticuloendothelial system (RES). The parasites are highly successful in their ability to grow and multiply in the very tissues of the vertebrate host which are responsible for reaction to invading organisms. Expectedly, such a location of Leishmania renders difficult a satisfactory approach to chemotherapy, and there is highly complex inter-play between parasites and cellular immune responses of the host. In the RES, the parasites lie within the host macrophage for at least part of their life cycle. Fusion of host cell secondary lysosomes with the parasitophorous vacuoles apparently occurs without preventing subsequent multiplication of the Leishmania. Such fusion may provide means for access for nutrients to the parasite, but also exposes the parasite to host antibodies and lysosomal enzymes.

In man, the result of successful invasion of the spleen and liver by Leishmania donovani most frequently is death. Scarring of the skin may be the sole manifestation of infection with Leishmania tropica and allied dermatotropic organisms (as, Leishmania aethiopica, L. mexican, L. peruviana, and L. Guyanensis). Intermediate in severity are invasions of muco-cutaneous tissues by Leishmania braziliensis. Unfortunately, relatively few drugs have been found to show appreciable anti-Leishmanial activity on screening, and fewer yet have merited trial in man. Antimony drugs are a mainstay for treatment, yet severe toxic side effects may occur, in particular, among poorly nourished patients. Of the antimonial drugs, one widely used in the clinic is the N-methyl glucamine salt of antimonic acid, frequently called meglumine antimonate. Toxicity of such drugs may affect the liver (hepatitis), kidneys (nephritis), or the heart (myocarditis). Of these toxic effects, myocarditis is the greatest and most common problem.

Pentamidine was originally shown to be useful for the treatment of trypanosomiasis and of more recent time, pentamidine, as indicated above, has been found to be extremely useful in the treatment of Pneumocystis carinii pneumonia, especially in patients suffering from the acquired immunodeficiency syndrome (AIDS). Pentamidine, but not its analogues, has heretofore been known to have some limited utility in the treatment of Leishmaniasis.

In view of the lack of a satisfactory agent for the treatment of Leishmaniasis, a need exists for a more-effective anti-Leishmanial agent having good therapeutic properties.

## Malaria

Human malaria is caused by species of parasitic organisms of the genus Plasmodium. It is transmitted by mosquitoes which ingest sexual forms of the parasite in blood meals. Sporozoite forms of the parasite develop in the mosquito and are transmitted to new host individuals bitten by the insect. The major human pathogen is Plasmodium falciparum.

Malaria is one of the most important health problems in underdeveloped, tropical countries. It is estimated that more than a billion people in the world inhabit areas in which malaria is transmitted. Although chloroquine has been used as an effective drug, this drug has some side effects, but more importantly, malarial parasites have acquired a resistance to chloroquine.

Thus, malaria has become an increasing problem in the tropical zones with the advent of chloroquine resistant strains of malaria parasites coupled with a decreased effectiveness of long acting insecticides such as DDT. The magnitude of the problem is reflected in the fact that malaria is the largest infectious disease in the world. Of the one billion people residing in malaria endemic areas, approximately 25 to 200 million people are diseased at any given time.

There are estimates of a million malaria deaths a year in Africa, chiefly among children under five. Even after surviving childhood infection, a large proportion of adults nonetheless remain susceptible to infection and show periodic parasitemia, even though their serum contains "protective" antiplasmodial antibodies. In hyperendemic areas of Africa, it is believed that nearly all residents harbor a continuous series of falciparum infections of low to moderate pathogenicity throughout their lives.

The problem of malarial infection has become even more serious as more strains of malaria have become resistant to the major anti-malaria drug chloroquine. More and more chloroquine resistant strains of

Plasmodium falciparum have emerged in Central and South America, Africa, and Southeast Asia.

Researchers have synthesized chemical variants of chloroquine to combat new resistant malaria strains; however, these strains have already become resistant to the new drugs. Recently a new drug, mefloquine, was introduced, but already resistant strains have appeared. A totally new drug having chemical properties different from chloroquine is needed to stem the increasing epidemic of resistant malaria strains.

Pentamidine has been known for decades and was originally shown to be useful for the treatment of trypanosomiasis. As detailed above, pentamidine has been found to be extremely useful in the treatment of Pneumocystis carinii pneumonia, especially in immunocompromised patients suffering from the acquired immunodeficiency syndrome (AIDS). Likewise, pentamidine, but not analogues thereof, has been known to have utility in the treatment of malaria.

It goes without saying that in view of the magnitude of malaria infection throughout the world, and the lack of a satisfactory agent for the treatment thereof, an urgent need exists for a more effective anti-Plasmodial agent having good therapeutic properties.

## SUMMARY OF THE INVENTION

### Pneumocystis carinii Pneumonia

In accordance with one aspect of the present invention, surprisingly, it has now been discovered that Pneumocystis carinii pneumonia may be effectively treated with certain compounds, as defined in Formula I:

I

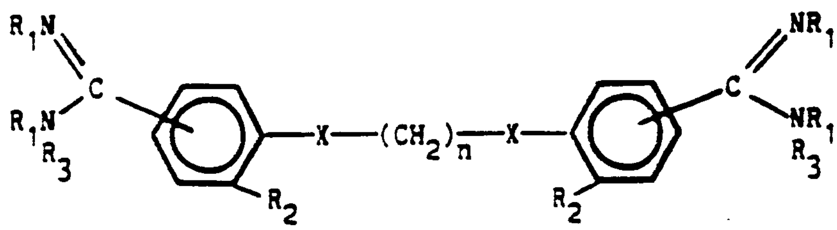

wherein each $R_1$ is H or two $R_1$ groups on the same amidine group together represent $-(CH_2)_m-$, wherein m = 2, 3, or 4; $R_2$ is H, $OCH_3$, $NO_2$ or $NH_2$; $R_3$ is H, $CH_3$, or $CH_2CH_3$; n = 2, 3, 4 or 5; and X is O, NH or S; provided that when both $R_1$ and $R_2$ are H and X = O, then n cannot equal 5.

Particularly preferred are those compounds of Formula I which have the para-amidine structure, as shown by Subformula Ia:

Ia

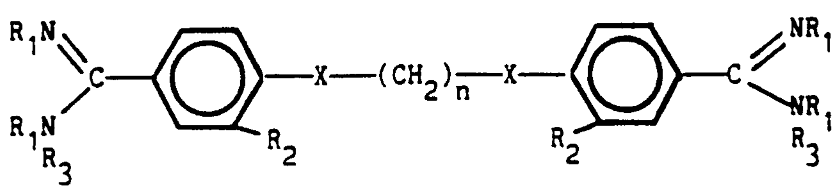

wherein $R_1$, $R_2$, $R_3$, X, m and n have the same meanings as for Formula I.

Many of the compounds which now have been found to be useful in the treatment of, or prophylaxis against, Pneumocystis carinii pneumonia are themselves new compounds. Such new compounds are defined by Formula II, as follows:

II 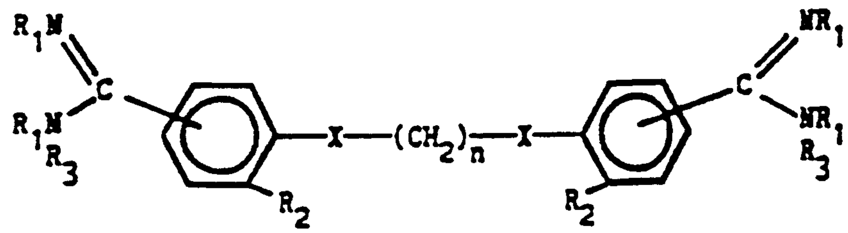

wherein each $R_1$ is H or two $R_1$ groups on the same amidine group together represent $-(CH_2)_m-$, wherein m = 2, 3 or 4; $R_2$ is H, $OCH_3$, $NO_2$ or $NH_2$; $R_3$ is H, $CH_3$ or $CH_2CH_3$, n = 2, 3, 4 or 5; and X is O, NH or S; with the provisos that when both $R_1$ and $R_2$ are H, then X is NH or S; and when $R_2$ is H and X is O, then two $R_1$ groups together represent $-(CH_2)_m-$ and n = 3 or 4; and when X is O or S and n = 5, then $R_1$, $R_2$ and $R_3$ are not H at the same time; and when two $R_1$ groups on the same amidine group together represent $-(CH_2)_3-$, X is S and n = 5, then $R_2$ and $R_3$ are not H at the same time; and when X is O, two $R_1$ groups on the same amidine group together represent $-(CH_2)_2-$ and n = 5, then $R_2$ and $R_3$ are not H at the same time.

Particularly preferred are those compounds of Formula II which have the para-amidine structure, as shown by Subformula IIa:

IIa 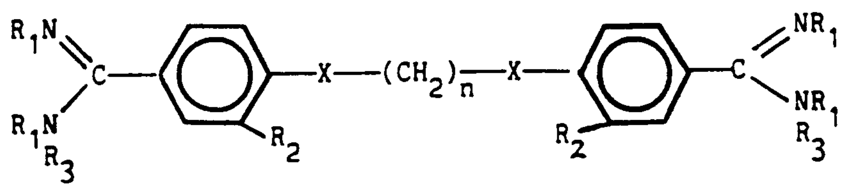

wherein $R_1$, $R_2$, $R_3$, X, m and n have the same meanings as for Formula II. Additionally, new compounds as otherwise defined in Formula II, but wherein n = 6 show efficacy against PCP, but have high toxicity.

## Giardiasis

It also has been discovered that Giardia lamblia may be treated with pentamidine and analogues thereof. Accordingly, the present invention provides compounds for use in preparing a pharmaceutical preparation for treating giardiasis. The treatment comprises administering to an afflicted host a therapeutically effective amount of compound having the structure of formula I, wherein X is O, NH or S; $R_1$ is H or two $R_1$ groups on the same amidine group together represent $-(CH_2)_m$, wherein m = 2, 3 or 4; $R_2$ is H, $NH_2$, $OCH_3$, Cl, or $NO_2$; $R_3$ is H, $CH_3$ or $CH_2CH_3$ and n = 2-6, or pharmaceutically acceptable salts thereof, or more preferably a compound of formula II, wherein X, $R_1$, $R_2$, $R_3$, m and n have the foregoing meanings, or a pharmaceutically acceptable salt thereof.

## Leishmaniasis

Surprisingly, it now has been discovered that leishmaniasis may be treated with certain pentamidine analogues. Accordingly, the present invention provides compounds for use in preparing a pharmaceutical formulation for treating leishmaniasis. The treatment comprises administering to an afflicted host a therapeutically effective amount of compound having the structure of formula I, wherein X is O, NH or S; $R_1$ is H or two $R_1$ groups on the same amidine group together represent $-(CH_2)_m$, wherein m = 2, 3 or 4; $R_2$ is H, $NH_2$, $OCH_3$, Cl, or $NO_2$; $R_3$ is H, $CH_3$ or $CH_2CH_3$ and n = 2-6, provided that when both $R_1$ and $R_2$ are H and X = O, then n cannot equal 5, or pharmaceutically acceptable salts thereof, or more preferably a compound of formula II, wherein X, $R_1$, $R_2$, $R_3$, m and n have the foregoing meanings, or a pharmaceutically acceptable salt thereof.

## Malaria

It now has been discovered that malaria may be treated with certain pentamidine analogues. Accordingly, the present invention provides compounds for use in a method for treating malaria which comprising administering to an afflicted host a therapeutically effective amount of compound having the structure of

formula I, wherein X is O, NH or S; $R_1$ is H or two $R_1$ groups on the same amidine group together represent $-(CH_2)_m$, wherein m = 2, 3 or 4; $R_2$ is H, $NH_2$, $OCH_3$, Cl, or $NO_2$; $R_3$ is H, $CH_3$ or $CH_2CH_3$ and n = 2-6, provided that when both $R_1$ and $R_2$ are H and X = O, then n cannot equal 5, or pharmaceutically acceptable salts thereof, or more preferably a compound of formula II, wherein X, $R_1$, $R_2$, $R_3$, m and n have the foregoing meanings, or a pharmaceutically acceptable salt thereof.

**Novel Compounds**

Generally, the present invention also provides pharmaceutical formulations comprising the aforementioned new compounds of Formula II (or preferably of Formula IIa), or pharmaceutically acceptable salts thereof, in physiologically acceptable carriers. Also, the present invention provides such new compounds or salts thereof which have been lyophilized and which may be reconstituted to form pharmaceutically acceptable formulations for administration, as by intravenous or intramuscular injection.

**Novel Compositions**

Further, the present invention provides the aforementioned compounds or salts thereof as formulations for administration, as by aerosolization into particles or droplets for inhalation.

## DETAILED DESCRIPTION OF THE INVENTION

**Novel Compounds**

The distinguishing structural features between the new compounds of the present invention and those of the prior art are quite apparent, and readily may be ascertained by comparing the structures of such compounds with the structure of pentamidine, which is shown in Formula III:

III

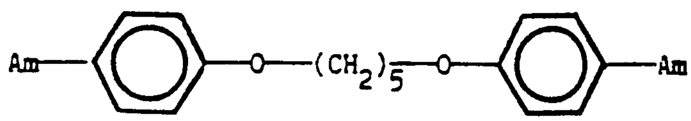

wherein Am represents an amidine group.

In one aspect of the present invention, the new compounds are distinguishable from pentamidine and previously known analogues thereof, by the presence of a nitrogen or sulfur atom, in place of the etheric oxygens in the group bridging the two aromatic nuclei. Such new compositions are represented by Formula II (or Subformula IIa), wherein X is NH or S. In such instances then the novel compounds have the structure of the following general Formula IVa or IVb, or preferably the specific para-amidine structure of Subformula IVc, or IVd:

IVa

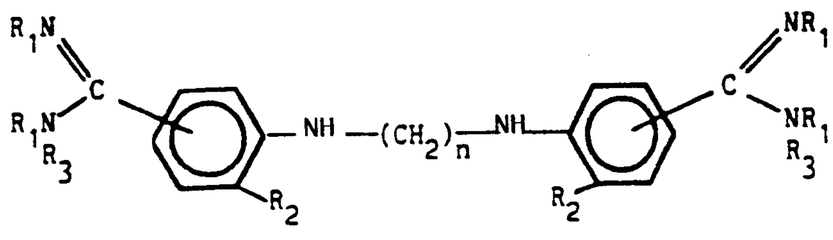

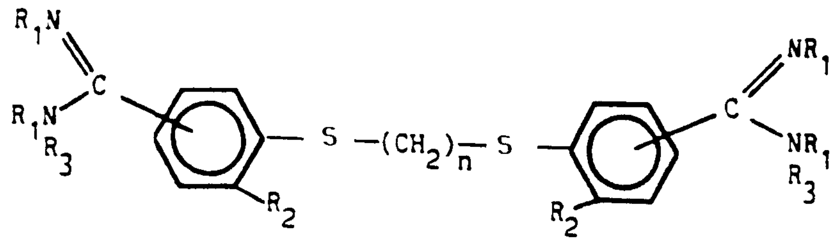

IVb

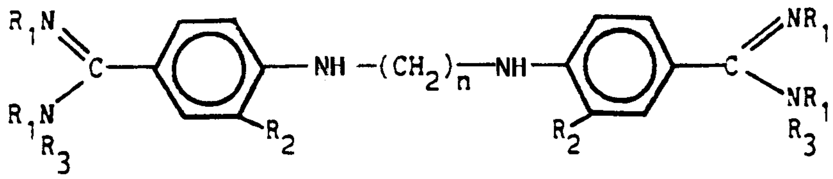

IVc

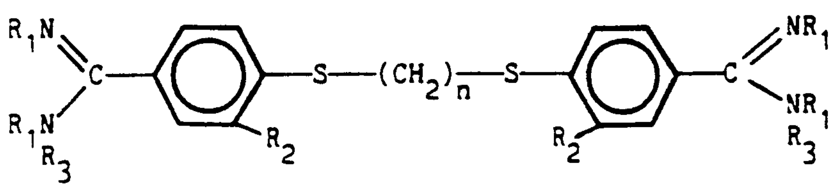

IVd

wherein each $R_1$ is H or two $R_1$ groups on the same amidine group together represent $-(CH_2)_m-$, wherein m is 2, 3 or 4; $R_2$ is H, $OCH_3$, $NH_2$ or $NO_2$; $R_3$ is H, $CH_3$ or $CH_2CH_3$; and n = 2, 3, 4 or 5. As indicated, no such compositions exist in the prior art in which the link between the two aromatic nuclei is by a group having the formula $-NH-(CH_2)_n-NH-$ or $-S-(CH_2)_n-S-$, and that feature alone distinguishes the compounds having the Formula IV from those of the prior art.

Another aspect of the present invention distinguishes new compounds of the present invention from the prior art through the presence of a methoxy, an amino or a nitro group on the two aromatic nuclei. Such compositions are represented in Formula II (or preferably in Formula IIa) when $R_2$ is $OCH_3$, $NH_2$ or $NO_2$ and may be represented specifically by Formula V or preferably by the para-amidine structure of Subformula Va:

V 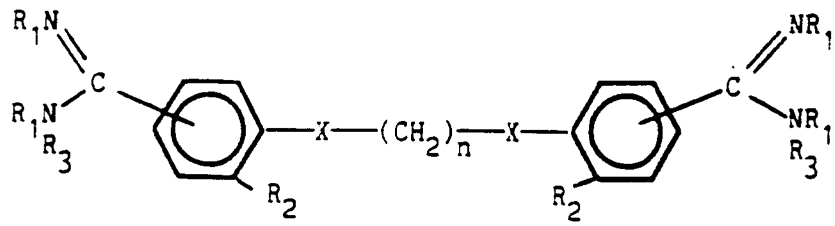

Va 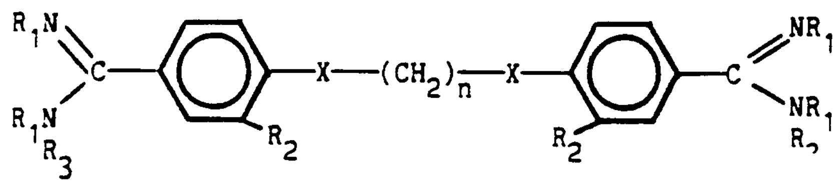

wherein each $R_1$ is H or two $R_1$ groups on the same amidine group together represent $-(CH_2)_m-$, wherein m is 2, 3 or 4; $R_2$ is $OCH_3$, $NH_2$ or $NO_2$; $R_3$ is H, $CH_3$, or $CH_2CH_3$; X is O, NH or S; and n = 2, 3, 4, or 5. Such compositions then are distinguished from pentamidine and analogues thereof through the presence of the methoxy, amino or nitro group, the methoxy and amino group having been found to increase the therapeutic efficacy of such compounds with respect to their activity against Pneumocystis carinii. The methoxy group in particular substantially increases the therapeutic efficacy of the compound.

In a further aspect of the present invention, certain of the new compounds are distinguished from the compounds of the prior art through the existence of closed ring derivatives of the amidine group, such as imidazoline rings, on both of the aromatic nuclei. The closed ring, such as imidazoline, is formed by bridging the nitrogen atoms on both of the amidine groups, through a $-(CH_2)_m$ group, such as $-CH_2CH_2-$. Referring to Formula II then, such compounds are represented when two $R_1$ groups on the same amidine group together represent $-(CH_2)_m-$ wherein m = 2, 3 or 4. Such compounds are unknown in the art when X is NH or S and/or when $R_2$ is $OCH_3$, $NH_2$ or $NO_2$. Further, such compounds are unknown in the art when X is O and n = 2, 3 or 4. The imidazoline compound is known, however, when X is O, $R_2$ is H, and n = 5. Said compound, however, is not known to have therapeutic efficacy against Pneumocystis carinii. However, the presence of the closed ring, such as an imidazoline group, on the new compounds of the present invention surprisingly has been found to substantially increase the therapeutic efficacy of the compounds with respect to the treatment of Pneumocystis carinii pneumonia. Such new imidazoline compounds are represented specifically by Formula VI or preferably by the para-imidazoline structure of Subformula VIa·

VI 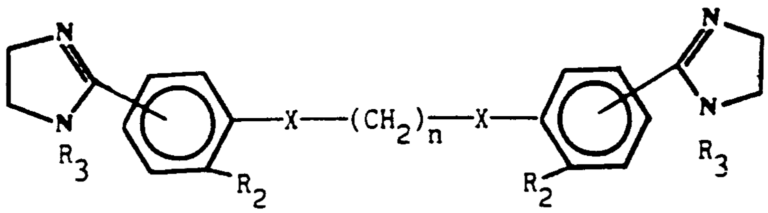

VIa 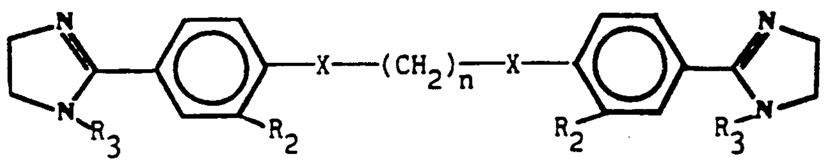

wherein $R_2$ is H, $OCH_3$, $NH_2$, or $NO_2$; $R_3$ is H, $CH_3$ or $CH_2CH_3$, X is O, NH or S; and n = 2, 3, 4 or 5 provided that when $R_2$ is H, n does not equal 5. The most preferred compound is represented by Subformula VIa when $R_2$ = $OCH_3$, $R_3$ = H, X = O, and n = 3.

**Treating and Prophylaxis, Pneumocystis carinii**

One especially important aspect of the present invention is the provision of compounds for use in a method for treating Pneumocystis carinii pneumonia. This comprises administering to a patient suffering from Pneumocystis carinii pneumonia, a therapeutically effective amount of a compound of Formula I or a pharmaceutically acceptable salt thereof. Heretofore, pentamidine was one of only a few compounds of a structure similar to the structure to the compounds of Formula I that has been known to be effective in the treatment of, or prophylaxis against Pneumocystis carinii pneumonia. The only other diamidines known to have some effectiveness against PCP are dibromopropamidine, stilbamidine and hydroxy-stilbamidine.

Besides providing a method for treating Pneumocystis carinii pneumonia, the present invention also provides compounds for use in a method for prophylaxis against Pneumocystis carinii pneumonia in an immuno compromised patient, such as one suffering from AIDS, who has had at least one episode of Pneumocystis carinii pneumonia, but who at the time of treatment is not exhibiting signs of pneumonia. As pneumocystis carinii pneumonia is an especially potentially devastating disease for immunocompromised patients, it is preferable to avoid the onset of Pneumocystis carinii pneumonia, as compared to treating the disease after it has become symptomatic. Accordingly, the present invention provides compounds for use in a method for the prophylaxis against Pneumocystis carinii pneumonia comprising administering to the patient a prophylactically effective amount of a compound of Formula I (and preferably of Subformula Ia) or a pharmaceutically acceptable salt thereof. The forms for administration of the compound or salt in accordance with this method may be the same as utilized for the purpose of actually treating a patient suffering from Pneumocystis carinii pneumonia.

An additional useful aspect of the present invention is a method for prophylaxis against even an initial episode of Pneumocystis carinii pneumonia in an immunocompromised patient who has never experienced an episode of Pneumocystis carinii pneumonia. In this respect, a patient who has been diagnosed as being immunocompromised, such as one suffering from AIDS or ARC (AIDS related complex), even before the onset of an initial episode of Pneumocystis carinii pneumonia, may avoid or delay suffering from the infection by having administered a prophylactically effective amount of a compound of Formula I (or preferably of Formula Ia) or a pharmaceutically acceptable salt thereof. The compound or salt may be administered in the same fashion as in the treatment of patients suffering from Pneumocystis carinii pneumonia.

## Method for Treating Giardiasis

The present invention provides also compounds for use in a new method for treating giardiasis by administering a therapeutically effective dose of a compound of formula I, above, or pharmaceutically acceptable salts thereof. Formula I encompasses pentamidine, along with various analogues or derivatives thereof, all of which are aromatic diamidines.

It has been found that with respect to compounds for use in the method of the present invention, treating Giardia lamblia with a compound of Formula I or preferably Formula II, or a pharmaceutically acceptable salt thereof, certain compounds appear to possess superior efficacy to others. Pentamidine, for example, has been found to be moderately effective against Giardia lamblia, as have most of the compounds within Formula I (or II) above. It was especially surprising to find that the most efficacious product within the scope of the present invention is a compound having a structure as defined by Formula II wherein $X = O$, $R_1$ and $R_3 = H$, $R_2 = OCH_3$ and $n = 3$. Very nearly identical in therapeutic efficacy against Giardia lamblia is the compound defined by Formula II wherein $X = NH$, $R_1$, $R_2$ and $R_3 = H$ and $n = 6$. When those compounds are compared to the aforementioned compounds presently of choice for use in treating giardiasis, it is seen that those compounds within the scope of the present invention essentially are as therapeutically efficacious as the current products for treating Giardia lamblia.

## Method of Treating Leishmaniasis

The present invention additionally provides compounds for use in a new method for treating Leishmaniasis by administering compounds of formula I, above, or pharmaceutically acceptable salts thereof. Formula I encompasses pentamidine, along with various analogues or derivatives thereof, all of which are aromatic diamidines.

It has been found that with respect to compounds for use in the method of the present invention, in treating Leishmania infection with a compound of Formula I (or preferably Formula II), or a pharmaceutically acceptable salt thereof, certain compounds appear to possess superior efficacy to others. Pentamidine, for example, has been found to be moderately effective against Leishmania as have most of the compounds within Formula I (or II) above. It was especially surprising to find that the most efficacious products within the scope of the present invention are a compound having a structure as defined by Formula II wherein $X = NH$; $R_1$, $R_2$, and $R_3 = H$, and $n = 5$, and a compound having the structure as defined by Formula II wherein $X = O$; $R_1$, $R_2$ and $R_3 = H$, and $n = 6$. Both compounds are structurally similar to pentamidine.

## Method for Treating Malaria

The present invention also provides compounds for use in a new method for treating malaria by administering compounds of formula I, above, or pharmaceutically acceptable salts thereof. Formula I encompasses pentamidine, along with various analogues or derivatives thereof, all of which are aromatic diamidines.

It has been found that with respect to compounds for use in the method of the present invention, treating malaria with a compound of Formula I (or preferably Formula II), or a pharmaceutically acceptable salt thereof, certain compounds appear to possess superior efficacy to others. Pentamidine, for example, has been found to be moderately effective against malaria, as have most of the compounds within Formula I (or II) above. It was especially surprising to find that the most efficacious product identified to date, within the scope of the present invention, is a compound No. 106 having a structure as defined by Formula II wherein $X = NH$; $R_1$, $R_2$ and $R_3 = H$; and $n = 5$. Such a compound is identical to pentamidine, but possesses nitrogen atoms in place of the bridging oxygen atoms found in pentamidine. It was also very surprising that such a compound was the most effective compound against both the chloroquine-resistant strain (W2) and the mefloquine-resistant strain (D6).

Very close in therapeutic efficacy against malaria are compound Nos. 110, 113 and 116.

## Dosages and Dosage Forms

Obviously, the therapeutically effective dosage of any specific compound will vary somewhat from compound to compound and patient to patient. As a general proposition, a dosage from about 0.1 to about 20 mg/kg will have therapeutic efficacy. However, toxicity concerns at the higher level may restrict the dosage to a lower level such as up to about 10 mg/kg, based upon the weight of free-base. Typically, a dosage from about 0.5 mg/kg to about 5 mg/kg will be employed. The duration for the treatment is usually

once per day for a sufficient length of time for the patient to become asymptomatic. Depending upon the severity of the infection in the individual patient, this may last anywhere from two to three weeks, or longer.

A compound of Formula I or II, or a pharmaceutically acceptable salt thereof, may be administered orally or through inhalation as a solid, or may be administered orally, through inhalation, intramuscularly, or intravenously, as a solution, suspension, or emulsion. Alternatively, the compound or salt may also be administered by inhalation, intravenously or intramuscularly as a liposomal suspension. When administered through inhalation the compound or salt should be in the form of a plurality of solid particles or droplets having a particle size from about 0.5 to about 5 microns, preferably from about 1 to about 2 microns.

## Novel Compositions

The present invention also provides new pharmaceutical compositions suitable for intravenous or intramuscular injection. The pharmaceutical compositions comprise a compound of Formula II (and preferably of Subformula IIa), or a pharmaceutically acceptable salt thereof, in any pharmaceutically acceptable carrier. If a solution is desired, water is the carrier of choice with respect to water-soluble compounds or salts. With respect to the water-insoluble compounds or salts, an organic vehicle, such as glycerol, propylene glycol, polyethylene glycol, or mixtures thereof, may be suitable. In the latter instance, the organic vehicle may contain a substantial amount of water. The solution in either instance may then be sterilized in any suitable manner, preferably by filtration through a 0.22 micron filter. Subsequent to sterilization, the solution may be filled into appropriate receptacles, such as depyrogenated glass vials. Of course, the filling should be done by an aseptic method. Sterilized closures may then be placed on the vials and, if desired, the vial contents may be lyophilized.

In addition to compounds of Formula II (and preferably of Subformula IIa) or their salts, the pharmaceutical compositions may contain other additives, such as pH adjusting additives. In particular, useful pH adjusting agents include acids or bases or buffers, such as sodium lactate, sodium acetate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multi- dose use. Of course, as indicated, the pharmaceutical compositions of the present invention may be lyophilized using techniques well known in the art.

In yet another aspect of the present invention, there is provided an injectable, stable, sterile composition comprising a compound of Formula II (and preferably of Subformula IIa), or a salt thereof, in a unit dosage form in a sealed container. The compound or salt is provided in the form of a lyophilizate which is capable of being reconstituted with a suitable pharmaceutically acceptable carrier to form a liquid composition suitable for injection thereof into man. The unit dosage form typically comprises from about 10 mg to about 10 grams of the compound or salt. When the compound or salt is substantially water-insoluble, a sufficient amount of emulsifying agent which is physiologically acceptable may be employed in sufficient quantity to emulsify the compound or salt in an aqueous carrier. One such useful emulsifying agent is phosphatidyl choline.

In accordance with the present invention, other pharmaceutical compositions may be prepared from the water-insoluble compounds of Formula II (and preferably of Subformula IIa), or salts thereof, such as aqueous based emulsions. In such an instance, the composition will contain a sufficient amount of a pharmaceutically acceptable emulsifying agent to emulsify the desired amount of the compound of Formula II (and preferably of Subformula IIa) or salt thereof. Particularly useful emulsifying agents include phosphatidylcholines, and lecithin.

Further, the present invention provides liposomal formulations of the compounds of Formula II (and preferably of Subformula IIa) and salts thereof. The technology for forming liposomal suspensions is well known in the art. When the compound of Formula II or salt thereof is an aqueous- soluble salt, using conventional liposome technology, the same may be incorporated into lipid vesicles. In such an instance, due to the water solubility of the compound or salt, the compound or salt will be substantially entrained within the hydrophilic center or core of the liposomes. The lipid layer employed may be of any conventional composition and may either contain cholesterol or may be cholesterol-free. When the compound or salt of interest is water-insoluble, again employing conventional liposome formation technology, the salt may be substantially entrained within the hydrophobic lipid bilayer which forms the structure of the liposome. In either instance, the liposomes which are produced may be reduced in size, as through the use of standard sonication and homogenization techniques.

Of course, the liposomal formulations containing the compounds of Formula II or salts thereof, may be lyophilized to produce a lyophilizate which may be reconstituted with a pharmaceutically acceptable carrier,

such as water, to regenerate a liposomal suspension.

In another aspect of the present invention, pharmaceutical formulations are provided which are suitable for administration as an aerosol, by inhalation. These formulations comprise a solution or suspension of the desired compound of Formula II (and preferably of Subformula IIa) or a salt thereof or a plurality of solid particles of the compound or salt. The desired formulation may be placed in a small chamber and nebulized. Nebulization may be accomplished by compressed air or by ultrasonic energy to form a plurality of liquid droplets or solid particles comprising the compounds or salts. The liquid droplets or solid particles should have a particle size in the range of about 0.5 to about 5 microns. The solid particles can be obtained by processing the solid compound of Formula II, or a salt thereof, in any appropriate manner known in the art, such as by micronization. Most preferably, the size of the solid particles or droplets will be from about 1 to about 2 microns. In this respect, commercial nebulizers are available to achieve this purpose.

Preferably, when the pharmaceutical formulation suitable for administration as an aerosol is in the form of a liquid, the formulation will comprise a water-soluble compound of Formula II (and preferably of Subformula IIa) or a salt thereof, in a carrier which comprises water. A surfactant may be present which lowers the surface tension of the formulation sufficiently to result in the formation of droplets within the desired size range when subjected to nebulization.

As indicated, the present invention provides both water-soluble and water-insoluble compounds and salts. As used in the present specification, the term "water-soluble" is meant to define any composition which is soluble in water in an amount of about 50 mg/mL, or greater. Also, as used in the present specification, the term "water-insoluble" is meant to define any composition which has solubility in water of less than about 20 mg/mL. For certain applications, water soluble compounds or salts may be desirable whereas for other applications water- insoluble compounds or salts likewise may be desirable.

## Synthesis Procedure

The compounds employed in the present invention, whether known compounds or novel compounds, may be synthesized in manners generally known and readily understood by those skilled in the art. Therefore, there is no need to explain in great detail the methodology used for the synthesis of most of those compounds. The following summary of the synthesis routes as employed is an aid to those skilled in the art in choosing the appropriate known synthesis procedure to employ for the respective classes of compounds.

In general, the several reaction schemes which may be employed to synthesize the compounds of the present invention are shown in Charts I-III. As shown in Chart I, the compounds of Formula I in which n = 2-5 (and for comparative purposes when n = 6), $R_1$ = H, X = O, and $R_2$ = H or $OCH_3$ may be prepared by alkylation of cyanophenol (with methoxy substitution when appropriate) with dibromoalkanes, to yield the corresponding cyano analogues to the compounds of Formula II, employing generally the procedure of Geratz et al., J. Med. Chem. 16: 970, 1973. The cyano analogue may then be subjected to Pinner's amidine synthesis to yield the desired products. Additionally, the imidate which is also obtained through the aforementioned alkylation reaction may be refluxed with ethylene diamine to yield the imidazoline products of the present invention which are represented by Formula I when two $R_1$ groups on an amidine group together represent $-CH_2CH_2-$. Further, the cyano compounds obtained as a result of the aforementioned alkylation reaction may be further reacted by nitrating said compounds using acetyl nitrate in trifluoroacetic acid, resulting in dinitrodicyano compounds which then may be converted to the corresponding amidines, using the aforementioned Pinner's amidine synthesis. The dinitrodiamidine compounds which result may be investigated for comparative purposes. Those dinitrodiamidine compounds may be further converted through catalytic reduction with $H_2$ and Pd/C to yield the corresponding diaminodiamidines useful in accordance with the present invention.

As shown in Chart II, the diazo derivatives which are represented by Formula I wherein X = NH and $R_2$ = H may be synthesized through a nucleophilic displacement reaction of 4-fluorobenzonitrile with diaminoalkanes, followed by the Pinner's amidine synthesis. Similarly, 4-chloro-3-nitrobenzonitrile may be reacted with diamino-alkanes to yield the corresponding cyano derivative. Conversion of that intermediate to the final product is then dependent upon the relative solubilities. In this respect reference is made to Chart III which shows the synthesis of the compound wherein X = NH, $R_2$ = $NH_2$ and n = 2, by reaction of 4-chloro-3-nitrobenzonitrile with a ten-fold excess of ethylene diamine at 25 degrees C to yield the corresponding mono-derivative which then may be catalytically reduced to form the corresponding amine, followed by a second nucleophilic displacement reaction with 4-chloro-3-nitrobenzonitrile to yield the corresponding dicyanomonoaminomononitrile which may then be subjected to Pinner's amidine synthesis and a final reduction to yield the desired diaminodiamidine product.

The compounds of Formula I wherein X = NH and $R_2 = NH_2$, with n = 4 or 6 likewise present a synthesis issue in that the intermediate cyano derivative obtained through the reaction of 4-chloro-3- nitrobenzonitrile with the corresponding diamino alkanes results in an intermediate which is not soluble in any appropriate solvent for conversion to the corresponding diamidine derivative. In such an instance, the nitro groups may be reduced to amino groups and then converted to the desired diamidines, as depicted in route 2 on Chart II. The otherwise identical compounds, but wherein n = 3 or 5, are slightly soluble in dioxane and therefore capable of being converted to diamidines directly, before reduction of the nitro groups to yield the final desired diaminodiamidine compounds.

As indicated, the compounds used in the present invention may be present as pharmaceutically acceptable salts. Such salts include the gluconate, lactate, acetate, tartrate, citrate, phosphate, borate, nitrate, sulfate, and hydrochloride salts.

The salts of the present invention may be prepared, in general, by reacting the amidine base compound with slightly in excess of two equivalents of the desired acid, in solution. After the reaction is complete, the salts are crystallized from solution by the addition of an appropriate amount of solvent in which the salt is insoluble.

The present invention will be further illustrated by the following non-limiting examples.

**Examples 1-44 (including comparative examples)**

Compounds having the structure represented by Formula I were synthesized in accordance with the appropriate procedures discussed previously. The reaction schemes shown in Charts I-III specifically identify the methodology used to synthesize the compounds of the respective examples. The compounds which were synthesized are shown in Table IA and the elemental analysis and melting points of the compounds are shown in Table IB. Most of the compounds were tested for toxicity in rats using standard laboratory procedure and the results of that toxicity testing are shown in Table XII. The compounds of Examples 31, 32, and 34 were not synthesized but may be synthesized in the general manner discussed previously.

**TESTING OF THE COMPOUNDS FOR THERAPEUTIC EFFICACY AGAINST Pneumocystis carinii**

**Induction and Treatment Of Pneumocystis carinii in Sprague-Dawley Rats**

Male Sprague-Dawley rats, barrier raised, non-certified virus free, weighing 150-200 grams were obtained from Hilltop Laboratories and housed individually. Animals were begun on a low (8%) protein diet (ICN Biomedicals, Cincinnati, OH) and drinking water containing tetracycline (0.5mg/ml) and dexamethasone (1.0mg/ml) immediately upon arrival. This treatment was given for the next 8 weeks, monitoring fluid intake daily and weighing animals weekly. Dilutions of the drinking solution were made when animals consumed too much fluid so as to prevent cortisone poisoning. At the beginning of the sixth week, animals were divided into groups of 8 animals each and the test compounds were administered daily by i.v. injection at a dose of 10 mg/kg, unless otherwise noted, for the next 14 days.

Animals were sacrificed at the end of the eighth week by chloroform inhalation and the left lung was removed aseptically and placed in sterile Hank's balanced salts solution without calcium or magnesium (HBSS-) for the ground tissue procedure. The right lung was inflated in situ with 10% formula and removed for histologic examination GMS staining. Tissue processing procedure

Rat lungs not immediately processed were quickly frozen and stored at -70 degrees C. Tissues were removed from the freezer when ready for processing and quickly defrosted in a 25 degrees C waterbath. The lungs were then cut into small pieces and ground through a #60 wire mesh with a glass pestle. The minced lungs were suspended in 10ml of HBSS- and vortexed for 30 seconds. The suspension was centrifuged at 60 X g for 10 minutes, discarding the pellet and transferring the supernatant fluid to another tube and centrifuging at 150 X g for 10 minutes to remove the remaining cellular debris. The supernatant from this centrifugation was then spun at 10,000 X g for 10 minutes to pellet the Pneumocystis organisms. This pellet was resuspended in 2ml of HBSS- for staining. Slides were prepared by placing a 10ul drop of lung suspension on a clean microscope slide and allowing the drop to air dry. The slides were then stained with cresyl echt violet (Kodak Chemicals, Rochester, NY) to demonstrate the cyst form of the organism.

**Statistical Studies**

A total of 20 high power microscopic fields were counted for each lung suspension and the mean number of cysts was calculated.

**RESULTS OF EXPERIMENTS**

**Table II**

One compound useful in the treatment of PCP in accordance with the present invention, Example 4, was tested in comparison with pentamidine in both the normal para-form as well as the meta-form. The compound of Example 4 differed from pentamidine (para) in that the group linking the two aromatic nuclei was a $-(CH_2)_3-$ group rather than the $-(CH_2)_5-$ group of pentamidine. The compound of Example 4 was found better at controlling the extent of the pcp infection than either para- or meta-pentamidine.

**Table III**

Two novel compounds of the present invention having the structure of Formula II (and of Subformula IIa) wherein the structure differs from that of pentamidine in one instance because $R_2$ is a methoxy group and in the other instance because $R_2$ is an amino group (Example 15 and 14, respectively) were tested in comparison with pentamidine. Also included in the study was a known compound wherein the structure differed from that of pentamidine in that n is 6, representing then hexamidine (Example 20). Surprisingly, it was found that all three variations in structure from that of pentamidine resulted in improved performance in treating **Pneumocystis carinii** pneumonia.

**Table IV**

A compound of Formula I (and of subformula Ia), butamidine (Example 9), was compared with pentamidine and two analogues thereof, the first within the scope of Formula II (and of Subformula IIa) wherein $R_2$ represents $NH_2$ (example 19) and the second within the scope of both Formulae I (and of Subformula Ia) and II (and of Subformula IIa) wherein $R_2 = NO_2$. In the case of the amino substituted compound (Example 19), the etheric oxygens of pentamidine (position X) were also replaced by nitrogen. Of great surprise was the finding that the butamidine was significantly better than pentamidine in controlling **Pneumocystis carinii** pneumonia. Both of the other compounds were better than the control but were not as good as pentamidine in controlling **Pneumocystis carinii** pneumonia.

**Table V**

Four compounds of Formula II (and of Subformula IIa, Examples 7, 8, 17, and 18) similar to pentamidine with $n = 3$-5, but having NH in place of O (position X) were tested in comparison to pentamidine and derivatives in which the chain length of the bridging group was $n = 6$, resulting in high toxicity. The new compound with nitro substitution ($n = 5$, Example 18) was better than the control but was less effective than pentamidine, while the new compounds of examples 7, 8, and 17 were better than or equal to pentamidine, with the $n = 3$ compound (Example 7) being most effective and the $n = 5$ compound (Example 17) being the least effective. The $n = 6$ derivatives were comparable to pentamidine but had toxicity concerns (See Table XIII).

**Table VI**

Four novel compounds of Formula II (and Subformula IIa, Examples 3, 5, 10, and 14) were tested in comparison with pentamidine, with respect to their effectiveness in treating Pneumocystis carinii pneumonia, the compounds all having amino ($NH_2$) substitution on the aromatic nuclei, as shown in Formula II (and Subformula IIa) wherein $R_2 = NH_2$. Further, in all instances the group linking the aromatic nuclei contained two etheric oxygens, as represented when $X = O$. The chain length of the bridging alkyl group was varied from 2 through 5 and for comparative purposes an additional compound, otherwise identical to the compounds within Formula II, was employed wherein the alkyl chain length was 6 (as represented when $n = 6$). The compounds within the scope of Formula II (and Subformula IIa) were all significantly better than the control with respect to the treatment Pneumocystis carinii pneumonia and were approximately comparable to pentamidine in efficacy. The compound of example 22 wherein n = 6 was significantly less effective than the other compounds or pentamidine in treating Pneumocystis carinii pneumonia and was toxic, see Table XIII.

**Table VII**

For comparative purposes, pentamidine and four analoques wherein the amidine group was in the meta position were analyzed for efficacy in treating Pneumocystis carinii pneumonia. The meta-amidine analogues had linking groups varying in carbon chain length from 3 to 6, as shown when n = 3-6. Although none of the meta amidines functioned as well as para-pentamidine with respect to therapeutic efficacy, they were all better than the control. Especially of interest is the fact that the meta-compounds having the shorter chain-length bridging groups (n = 3,4) were better than the meta-form of pentamidine. Also for comparative purposes, a blocked amidine, otherwise identical to pentamidine was employed and found to be comparable to or perhaps slightly better than pentamidine with respect to therapeutic efficacy.

**Table VIII**

Three compounds within Formula II (and Subformula IIa, Examples 6, 11 and 15) containing methoxy groups were compared at 5 mg/kg to pentamidine with respect to therapeutic efficacy in treating Pneumocystis carinii pneumonia. Said compounds are represented in Formula II (and Subformula IIa) when $R_2 = OCH_3$. Further, those specific compounds contained oxygen in the group bridging the two aromatic nuclei, as shown in Formula II when X = O. The length of the bridging carbon chain varied from 3 through 5 as shown when n = 3-5. Also included in the study were two compounds wherein chlorine atoms were substituted on the aromatic nuclei, as would be represented by Formula IIa if $R_2 = Cl$, which were slightly better than the control but much worse at a dose of 2.5 mg/kg than pentamidine in treating Pneumocystis carinii pneumonia. The compounds containing the methoxy group were significantly better, at one half the dose, than the control and, depending on chain length, worse than, equal to, or significantly better than pentamidine in treating Pneumocystis carinii pneumonia, at one half the dose of pentamidine. The shorter the chain length (n = 3) the better the efficacy for such methoxy substituted compounds, with efficacy diminishing with increased chain length.

**Table IX**

Four compounds within the scope of Formula II (Examples 33, 41, 42, and 44) were compared against pentamidine with respect to therapeutic efficacy in treating Pneumocystis carinii pneumonia. In one instance (Example 33) the novel compound of the present invention contained an amino substituent on the aromatic nuclei ($R_2 = NH_2$) and had nitrogen atoms in the group bridging the two aromatic nuclei (X = NH), with short bridging alkyl chain length (n = 2). That compound was better than the control, but worse than pentamidine. Three compounds wherein the amidine nitrogen groups had been linked through an ethylene bridge, to produce imidazolines were also compared at 2.5 mg/kg (Examples 41, 42, and 44). The compound of Example 44 having methoxy substitution on the aromatic nuclei ($R_2 = OCH_3$) and as the group bridging the aromatic nuclei, $-O(CH_2)_3O-$, as represented when X = O and n = 3, was found to be very effective in treating Pneumocystis carinii, being much better than pentamidine at only one fourth the dose. Two similar compounds were also tested which had methyl substituents on the imidazoline groups. The presence of such methyl groups were found to significantly decrease efficacy of the compound, while still being better than the control. Compound 44 represents the most preferred embodiment of the present invention in that the efficacy in treating Pneumocystis carinii pneumonia is very good at one fourth the dose level of pentamidine.

**Table X**

Six new compounds within the scope of Formula IIa (Examples 35, 36 37, 38, 40 and 43) were tested for therapeutic efficacy against Pneumocystis carinii pneumonia. Two new compounds (Examples 36 and 37) had amino substitution ($R_2 = NH_2$) on the aromatic nuclei, along with nitrogen atoms in the group bridging the aromatic nuclei (X = NH). Those compounds were compared to similar new compounds in which the substituents on the aromatic nuclei were nitro groups (Examples 35 - tested at 5 mg/kg - and 38 - tested at 2.5 mg/kg), the chain length of the bridging group being varied from 3 to 4 (n = 3-4). The amino substituted compounds were found to be better than the nitro substituted compounds, although the amino

substituted compounds were tested at a higher dosage level. Also, two compounds within Formula IIa (Examples 40 and 43) having imidazoline groups (wherein two $R_1$ groups equal $-CH_2CH_2-$) were tested, one compound (Example 43) having methoxy substitution on the aromatic nuclei ($R_2 = OCH_3$), with the group bridging the aromatic nuclei being $-O(CH_2)_5O-$ and in the other instance (Example 40) no substitution on the aromatic nuclei, with the bridging group being $-O(CH_2)_4O-$. Both such novel compounds were found to be significantly better than the control, even though the compound of Example 43 was tested at a lower level of 5 mg/kg.

**Table XI**

To compare the efficacy of butamidine (R1 = H, R2 = H, X = O, n = 4) against pentamidine, a logarithmic comparison was undertaken wherein both butamidine and pentamidine were tested for efficacy against Pneumocystis carinii pneumonia at dosages of 10 mg/kg, 1 mg/kg and 0.1 mg/kg. The butamidine was significantly better than pentamidine at the 10 mg/kg dosage level, confirming the results reported earlier in Table IV. However, when the dosage level was reduced to 1 mg/kg or 0.1 mg/kg, there was essentially no difference in the two compounds.

**Summary**

The composite results of the foregoing in vivo testing is set forth in Table XII. Although such compilations are of somewhat uncertain significance due to variations in control results from experiment to experiment, it still serves as a useful tool and fully summarizes the utility of the present invention.

**Examples 45-80**

**Testing Against Giardia lamblia**

The present invention will be further described in accordance with the following non-limiting examples. Examples 45-88 (including comparative examples).

Compounds falling within the scope of Formula I (and II) were obtained, having the structures identified in Tables XIV through XVI. To test those compounds against Giardia lamblia the following general procedure was employed. Axenic Culture of Giardia lamblia Trophozoites.

Giardia lamblia WB strain (ATCC #30957) was grown in filter sterilized TY-S-33 medium (Diamond et al., 1978) modified by the addition of bile (Keister, 1983) and containing 10% heat-inactivated fetal bovine serum and 50 ug/ml ampicillin (Sigma) and 50 ug/ml gentamicin sulfate (Sigma). Stock cultures of trophozoites were grown in 13 X 100 mm screw-capped borosilicate glass tubes at 37 degrees C. Organisms were subcultured every 72 hours by chilling the culture tube in an ice-water bath for 5 minutes. The trophozoites were dislodged from the glass by inverting the chilled tube vigorously. The number of organisms per ml was determined by placing 10 ul of the suspension on a hemocytometer. Approximately 5 X 104 organisms from a logarithmically growing culture were transferred into fresh media.

**Chemotherapeutic Agents**

Pentamidine and the analogs of pentamidine used in this study were synthesized using the procedures detailed previously. The metronidazole, quinacrine hydrochloride and furazolidone were obtained from Sigma. Stock solutions of 2 mM in modified TYI-S-33 medium were made and further diluted in medium for use in the assays of drug sensitivity. Microculture of Giardia lamblia

Giardia lamblia trophozoites were obtained from logarithmically growing stock cultures. Into the wells of a 96 well U-bottom tissue culture plate (Costar) was placed 100 ul of modified TYI-S-33 medium containing 2.5 X 104 trophozoites. The tissue culture plate was maintained in an anaerobic environment by placing it inside a plastic box modified to allow nitrogen to be passed through the box via inlet and outlet tubing which could be clamped shut. Nitrogen was passed through the box for 2 minutes and the container was placed in a 37 degree C incubator gassed with 5% CO2 in air. $^3$H-thymidine Incorporation Assay of Viability

Varying concentrations of $^3$H-thymidine (0.5, 1.0, 1.5 and 2.0 uCi/well) were added to wells containing 2.5 X 104 Giardia lamblia trophozoites at 2 hour intervals so that it might remain in contact with the trophozoites for 2 to 24 hours. After 24 hours of incubation, the cells were harvested with a multimash cell harvester by washing vigorously with ice-cold Hank's Balanced Salt Solution. Samples were collected on Whatman glass microfiber paper. The paper was air dried, samples were placed in 7 ml scintillation vials

and the incorporation of $^3$H-thymidine was determined by liquid scintillation counting. Mean values were calculated from 5 replicates for each time point and concentration. These data were used to determine the optimum time and concentration of $^3$H-thymidine to be used in the assay of drug sensitivity.

## Drug Sensitivity Assay

One hundred ul of modified TYI-S-33 medium containing 2.5 X 104 trophozoites was placed into each well of a 96-well U-bottom plate. The plates were incubated under anaerobic conditions for 24 hours. After 24 hours, the compounds to be tested were prepared from the 2 mM stock solutions and 100 ul of each solution was added to yield concentrations of 1, 10, 100 and 1000 uM in the wells. Ten ul of a 150 uCi/ml solution of $^3$H-thymidine (SA = 10 Ci/mmole) in modified TYI-S-33 was added 6 hours after the addition of drug to yield a final concentration of 1.5 uCi/well. After an additional 18 hours of incubation the cells were harvested. Five replicates were run for each drug concentration and the mean values determined. Non-specific binding of the $^3$H-thymidine to the microfiber paper was determined through the use of control wells to which no organisms were added. The activities of compounds tested were determined by comparing the incorporation of $^3$H-thymidine in the wells to which the compounds were added to that of wells of drug free controls. The drug concentration required to inhibit 50% incorporation of $^3$H-thymidine ($IC_{50}$) was determined (Chou, 1974).

## TEST RESULTS

The results of employing the foregoing procedure to determine the efficacy of compounds within the scope of Formula I or II above in treating Giardia lamblia are contained in Tables XIV-XVI. From those tables it is quite apparent that the very short chain length bridging group wherein n = 2 is the least efficacious, at least, when $R_2 = NH_2$. However, when n = 3 very effective results are obtained, whether $R_2$ = H, $NH_2$ or $OCH_3$. However, when $R_2 = OCH_3$, and $R_1$ and $R_3$ = H, the most beneficial result is obtained. It is certainly interesting to note then that the second best compound for treating Giardia lamblia in accordance with the practice of the present invention is defined by Formula I wherein X = NH, $R_1$, $R_2$ and $R_3$ = H, and n = 6, showing a relatively long group bridging the two aromatic nuclei.

From Tables XIV and XV it is seen that the meta amidines are comparable to the para-amidines, although somewhat lesser in activity, when the length of the group bridging the aromatic nuclei is in the range of n = 4-6. When n = 3, the meta amidine is much worse than its counterpart para-amidine.

From Table XVI it can be seen that the compounds of Formula II wherein the amidine groups have been converted to imidazolines are as a whole slightly less efficacious than their simple amidine counter parts. Also from Table XVI it is quite apparent that substitution on the imidazoline group, as with a methyl group, leads to drastically reduced efficacy. Table XVII contains efficacy data for three of the compounds currently of choice for treating Giardia lamblia. When one compares the efficacy of those compounds with the efficacy data of the most preferred compounds of the present invention, it can be seen that very little if any efficacy difference exists.

## Examples 81-98

## Testing Against Leishmania

The present invention will be further described in accordance with the following non-limiting examples. Examples 81-98

Compounds falling within the scope of Formula I (and II) were obtained, having the structures identified in Tables XVIII through XX. To test those compounds against Leishmania the following general procedure was employed.

## Chemotherapeutic Agents

Pentamidine and the analogs of pentamidine used in this study were synthesized using the procedures detailed above.

**In Vitro Screening For Anti-Leishmanial Activity**

Leishmania mexicana amazonensis, strain MHOM/BR/73/M2269 (WR669), promastigotes were grown to early log phase in Schneider's Drosophila Medium (GIBCO) supplemented with 20% heat-inactivated fetal bovine serum (GIBCO) and 100 ug/ml gentamicin sulfate [Parasitology 76:309 (1978). The assay medium was Schneider's plus 10% fetal bovine serum. Serial dilutions of the compounds of use in the present invention were suspended in the assay medium and prepared in duplicate rows of a 96-well microtiter plate. Parasite suspension (200 ul) at 2.5 x 106 cells/ml was added to each well and each plate was sealed and incubated under air at 25 degrees C. After 24 hours, methyl-$^3$H thymidine (20 Ci/mmol) was added to yield 1-2 uCi/well. After an additional 18 hours, the cells were harvested with a Skatron cell harvester onto glass microfiber filters. The filter disks were washed and dried and counted using a Beckman LS3801 scintillation counter. The data on uptake of $^3$H-thymidine was fitted to a logistic-logarithmic concentration response function by a non-linear regression method and the drug concentrations required to inhibit 50% incorporation of $^3$H-thymidine were determined [Antimicrob. Agents Chemother. 16:710 (1979); Exper. Parasitol. 66:86 (1988). The results are set forth in Tables XVIII-XX.

**TEST RESULTS**

The results of employing the foregoing procedure to determine the efficacy of compounds within the scope of Formula I (or II) above in treating Leishmania are contained in Tables XVIII-XX which show that para-amidines, meta-amidines, and para- imidazolines all have anti-Leishmanial activity. Compound Nos. 88 and 89 showed a high-degree of anti-Leishmanial activity. Compound 88 is identical to pentamidine except that it has nitrogen atoms in place of the bridging oxygen atoms. Compound 89 (hexamidine) is identical to pentamidine except that it contains one more methylene bridging group.

From Table XIX it is seen that the meta-amidines are comparable to the para-amidines, although they show a somewhat substantially lessened activity, in comparison with the para-amidines.

From Table XX it can be seen that the compounds of Formula II wherein the amidine groups have been converted to imidazolines are as a whole essentially as efficacious as their simple amidine counterparts.

**Examples 99-116**

**Plasmodium Activity**

The invention is further illustrated accordance with the following non-limiting examples.

Compounds falling within the scope of Formula I (and II) were obtained, having the structures identified in Tables XXI through XXIII. To test those compounds for efficacy in treating malaria the following in vitro general procedure was employed.

**Chemotherapeutic Agents**

Pentamidine and the analogs of pentamidine used in this study were synthesized using the procedures detailed above.

**Drug Sensitivity Assay**

Plasmodium falciparum strains W2 (chloroquine-resistant, mefloquine-sensitive) and D6 (mefloquine-resistant, chloroquine-sensitive) were cultured in a medium consisting of RPMI-1640, 25 mM HEPES, 25 mM $NaHCO_3$, and 10% (v/v) fresh frozen human plasma. Into the wells of a 96 well microtiter plate there was placed 200 ul per well of a 1.5% erythrocyte suspension containing 0.2% to 0.4% parasitized erythrocytes containing serial dilutions of the test compounds. The plate was then placed in an anaerobic chamber, flushed with nitrogen gas and incubated at 37 degrees C. After 24 hours, 25 ul per well of $^3$H-hypoxanthine (1 mCi/ml) were added and the plate reincubated as above. After 42 hours, the cells were harvested with a multimash-type cell harvester onto glass microfiber paper. A scintillation counter was used to determine the incorporation of $^3$H-hypoxanthine. The activities of the compounds tested were determined by comparing the incorporation of $^3$H-hypoxanthine to drug concentration using computerized non-linear regression analysis. [Antimicrob. Agents Chemother. 16:710, 1979; Am.J.Trop.Med.Hygiene 34:209, 1985. The results are shown in Tables XIX-XXI.

## TEST RESULTS

The results of employing the foregoing procedure to determine the efficacy of compounds within the scope of Formula I (or II) above in treating malaria are contained in Tables XXI-XXIII, which show that the para-amidines, meta-amidines, and para-imidazolines all have anti-plasmodial activity. The best compound for treating both strains of Plasmodium falciparum in accordance with the practice of the present invention is compound No. 106, defined by Formula I wherein X = NH, $R_1$, $R_2$ and $R_3$ = H, and n = 5.

From Table XXII it is seen that the meta-amidines are comparable to the para-amidines, although on the average they are somewhat lesser in activity than the para-amidines.

From Table XXIII it can be seen that the compounds of Formula II wherein the amidine groups have been converted to imidazolines are as a whole essentially as efficacious as their simple amidine counterparts.

Table IA

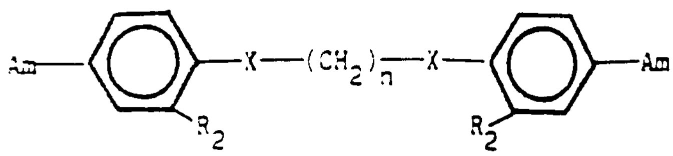

| # | X | n | $R_2$ |
|---|---|---|---|
| 1. | O | 2 | H |
| 2. | O | 2 | $NO_2$ |
| 3. | O | 2 | $NH_2$ |
| 4. | O | 3 | H |
| 5. | O | 3 | $NH_2$ |
| 6. | O | 3 | $OCH_3$ |
| 7. | NH | 3 | H |
| 8. | NH | 4 | H |
| 9. | O | 4 | H |
| 10. | O | 4 | $NH_2$ |

TABLE IA (continued)

| # | X | n | $R_2$ |
|---|---|---|---|
| 11. | O | 4 | $OCH_3$ |
| 12. | O | 5 | H |
| 13. | O | 5 | $NO_2$ |
| 14. | O | 5 | $NH_2$ |
| 15. | O | 5 | $OCH_3$ |
| 16. | O | 5 | Br |
| 17. | NH | 5 | H |
| 18. | NH | 5 | $NO_2$ |
| 19. | NH | 5 | $NH_2$ |
| 20. | O | 6 | H |
| 21. | O | 6 | $NO_2$ |
| 22. | O | 6 | $NH_2$ |
| 23. | NH | 6 | $NH_2$ |
| 24. | O | 4 | Cl |
| 25. | O | 5 | Cl |

TABLE IA (continued)

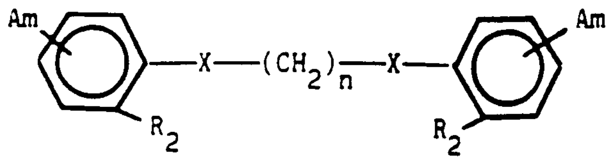

| # | X | n |
| --- | --- | --- |
| 26. | O | 3 |
| 27. | O | 4 |
| 28. | O | 5 |
| 29. | O | 6 |

TABLE IA (continued)

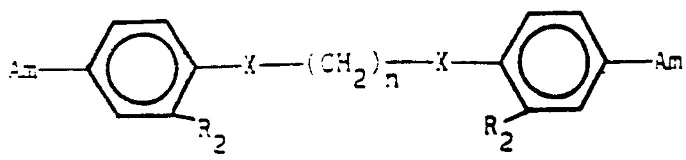

| # | X | n | $R_2$ |
|---|---|---|---|
| 31. | NH | 2 | H |
| 32. | NH | 2 | $NO_2$ |
| 33. | NH | 2 | $NH_2$ |
| 34. | O | 3 | $NO_2$ |
| 35. | NH | 3 | $NO_2$ |
| 36. | NH | 3 | $NH_2$ |
| 37. | NH | 4 | $NH_2$ |
| 38. | O | 4 | $NO_2$ |
| 39. | NH | 6 | H |

TABLE IA (continued)

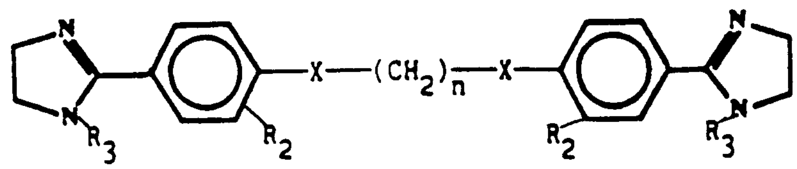

| # | X | n | $R_2$ | $R_3$ |
| --- | --- | --- | --- | --- |
| 30. | O | 5 | H | H |
| 40. | O | 4 | H | H |
| 41. | O | 4 | H | $CH_3$ |
| 42. | O | 5 | H | $CH_3$ |
| 43. | O | 5 | $OCH_3$ | H |
| 44. | O | 3 | $OCH_3$ | H |

TABLE IB

ELEMENTAL ANALYSIS AND MELTING POINTS OF EXAMPLES 1-44

| Cmpd. Number | Analyzed Formula | Elemental Analysis Calculated C | H | N | Found C | H | N | M.P. | Yield |
|---|---|---|---|---|---|---|---|---|---|
| 1 | $C_{16}H_{18}N_4O_2$/2HCl | 49.14 | 5.72 | 14.33 | 49.18 | 5.75 | 14.28 | >300 | 6% |
| 2 | $C_{16}H_{16}N_6O_6$/1.1HCl/1.3$H_2O$ | 42.53 | 4.39 | 18.60 | 42.83 | 4.01 | 17.07 | 287 | 39% |
| 3 | $C_{16}H_{20}N_6O_2$/4HCl/0.4$H_2O$ | 39.92 | 5.19 | 17.46 | 40.21 | 5.46 | 17.08 | >300 | 26% |
| 4 | $C_{17}H_{20}N_4O_2$/2HCl/1.3$H_2O$ | 49.96 | 6.07 | 13.71 | 49.96 | 6.10 | 13.59 | Dec at 144-145 | 82% |
| 5 | $C_{17}H_{22}N_6O_2$/4HCl/0.6EtOH | 40.81 | 5.98 | 15.69 | 40.72 | 5.62 | 15.31 | Dec at 273 | 6% |
| 6 | $C_{19}H_{24}N_4O_4$/2HCl | 51.24 | 5.88 | 12.58 | 51.04 | 5.91 | 12.50 | 293 | 76% |
| 7 | $C_{17}H_{22}N_6$/2HCl | 53.27 | 6.31 | 21.92 | 53.11 | 6.39 | 21.96 | 298-300 | 22% |
| 8 | $C_{18}H_{24}N_6$/2HCl/0.5$H_2O$ | 53.20 | 6.70 | 20.68 | 53.04 | 6.73 | 20.54 | >300 | 43% |
| 9 | $C_{18}H_{22}N_4O_2$/2HCl/0.4$H_2O$ | 53.18 | 6.15 | 13.78 | 53.41 | 6.21 | 13.41 | >300 | 95% |
| 10 | $C_{18}H_{24}N_6O_2$/4HCl/1$H_2O$ | 41.55 | 5.81 | 16.15 | 41.64 | 5.84 | 16.08 | 285 | 18% |
| 11 | $C_{20}H_{26}N_4O_4$/2HCl/1.2$H_2O$ | 49.94 | 6.37 | 11.65 | 49.98 | 6.29 | 11.61 | 297-299 | 70% |
| 12 | $C_{19}H_{24}N_4O_2$/2HCl/1$H_2O$ | 52.90 | 6.54 | 12.99 | 52.66 | 6.29 | 12.92 | 247 | 86% |
| 13 | $C_{19}H_{22}N_6O_6$/2HCl | 45.34 | 4.81 | 16.70 | 45.22 | 4.86 | 16.64 | 255 | 74% |
| 14 | $C_{19}H_{26}N_6O_2$/4HCl/1.6$H_2O$ | 41.86 | 6.14 | 15.42 | 41.83 | 6.12 | 15.37 | 270 | 40% |
| 15 | $C_{21}H_{28}N_4O_4$/2HCl/2$H_2O$ | 49.51 | 6.73 | 11.00 | 49.57 | 6.74 | 10.99 | 258-259 | 63% |

TABLE IB (continued)

ELEMENTAL ANALYSIS AND MELTING POINTS OF EXAMPLES 1-44

| Cmpd. Number | Analyzed Formula | Elemental Analysis: Calculated C | Calculated H | Calculated N | Found C | Found H | Found N | M.P. | Yield |
|---|---|---|---|---|---|---|---|---|---|
| 16 | $C_{19}H_{22}N_4O_2Br_2/2HCl/1H_2O$ | 38.73 | 4.45 | 9.51 | 38.84 | 4.64 | 9.46 | 254-255 | 58% |
| 17 | $C_{19}H_{26}N_6 2HCl/1.33H_2O$ | 52.42 | 7.10 | 19.30 | 52.32 | 7.08 | 19.17 | 295 | 48% |
| 18 | $C_{19}H_{24}N_8O_4/2HCl/2H_2O$ | 42.71 | 5.76 | 20.12 | 43.08 | 5.41 | 20.07 | 293-294 | 39% |
| 19 | $C_{19}H_{28}N_8/4HCl/2H_2O$ | 41.47 | 6.59 | 20.36 | 41.60 | 6.55 | 20.42 | 300-303 | 16% |
| 20 | $C_{20}H_{26}N_4O_2/2HCl/2.5H_2O$ | 50.91 | 7.32 | 11.31 | 50.94 | 7.05 | 11.31 | 252 | 69% |
| 21 | $C_{20}H_{24}N_6O_6/2HCl$ | 46.43 | 5.07 | 16.24 | 46.51 | 5.05 | 16.14 | Dec at 287-288 | 35% |
| 22 | $C_{20}H_{28}N_6O_2/4HCl/0.6H_2O$ | 45.49 | 6.45 | 15.01 | 45.13 | 6.84 | 14.61 | Dec at 290 | 18% |
| 23 | $C_{20}H_{30}N_8/4HCl/1.7H_2O$ | 42.97 | 6.74 | 20.05 | 43.05 | 6.76 | 19.97 | Dec at 285 | 18% |
| 24 | $C_{18}H_{18}N_4O_2/4HCl$ | 46.18 | 4.74 | 11.97 | 46.17 | 4.79 | 11.90 | >300 | 43% |
| 25 | $C_{19}H_{20}N_4O_2/4HCl/1.1H_2O$ | 45.46 | 5.26 | 11.16 | 45.49 | 5.23 | 10.98 | 247-248 | 55% |
| 26 | $C_{17}H_{20}N_4O_2/2HCl/1.9H_2O$ | 48.67 | 6.20 | 13.35 | 48.64 | 6.24 | 13.31 | 300 | 42% |
| 27 | $C_{18}H_{22}N_2O_2/2HCl/1.8H_2O$ | 50.07 | 6.44 | 12.98 | 50.10 | 6.46 | 12.97 | 257 | 68% |
| 28 | $C_{19}H_{24}N_4O_2/2HCl$ | 55.21 | 6.34 | 13.55 | 55.47 | 6.51 | 13.15 | 133-134 | 51% |
| 29 | $C_{20}H_{26}N_4O_2/2HCl/0.3H_2O$ | 55.51 | 6.66 | 12.95 | 55.45 | 6.67 | 12.88 | 268 | 64% |
| 30 | $C_{23}H_{28}N_4O_2/2.2H_2O$ | 54.70 | 6.87 | 11.09 | 54.69 | 6.87 | 11.06 | 147 | 81% |

TABLE IB (continued)

ELEMENTAL ANALYSIS AND MELTING POINTS OF EXAMPLES 1-44

| Cmpd. Number | Analyzed Formula | Elemental Analysis Calculated C | H | N | Found C | H | N | M.P. | Yield |
|---|---|---|---|---|---|---|---|---|---|
| 31 | Not Synthesized | | | | | | | | |
| 32 | Not Synthesized | | | | | | | | |
| 33 | $C_{16}H_{22}N_8$/4HCl/1.6$H_2O$/0.4EtOH | 38.84 | 6.13 | 21.57 | 38.83 | 6.00 | 21.81 | >300 | 27% |
| 34 | Not Synthesized | | | | | | | | |
| 35 | $C_{17}H_{20}N_8O_4$/2HCl/1$H_2O$ | 41.56 | 4.92 | 22.81 | 41.71 | 4.89 | 22.73 | Dec at 295 | 58% |
| 36 | $C_{17}H_{24}N_8$/4HCl/1.1$H_2O$/0.3EtOH | 40.66 | 6.20 | 21.55 | 40.29 | 6.29 | 21.21 | 229 | 10% |
| 37 | $C_{18}H_{26}N_8$/4HCl | 43.21 | 6.04 | 22.40 | 43.30 | 6.09 | 22.36 | Dec at 274-275 | 27% |
| 38 | $C_{18}H_{20}N_6O_6$/2HCl | 44.18 | 4.53 | 17.18 | 44.08 | 4.55 | 17.10 | 298 | 26% |
| 39 | $C_{20}H_{28}N_6$/2HCl/0.3$H_2O$ | 55.76 | 7.16 | 19.15 | 55.80 | 7.17 | 19.44 | 300 | 54% |
| 40 | $C_{22}H_{26}N_4O_2$/2HCl/2.2$H_2O$ | 53.81 | 6.65 | 11.41 | 53.80 | 6.70 | 11.36 | 249-250 | 76% |
| 41 | $C_{24}H_{30}N_4O_2$/1HCl/1.5$H_2O$ | 61.33 | 7.29 | 11.92 | 61.17 | 7.40 | 11.75 | 203-205 | 15% |
| 42 | $C_{25}H_{32}N_4O_2$/2HCl/1$H_2O$ | 58.71 | 7.09 | 10.95 | 58.73 | 7.16 | 10.88 | 203-204 | 19% |
| 43 | $C_{25}H_{32}N_4O_4$/2HCl/2.5$H_2O$ | 52.63 | 6.89 | 9.82 | 52.47 | 6.85 | 9.97 | 175 | 70% |
| 44 | $C_{23}H_{28}N_4O_4$/1.75HCl/2.00$H_2O$ | 52.69 | 6.49 | 10.69 | 52.59 | 6.38 | 10.46 | 252 | 62% |

TABLE II

Extent of Disease by Histologic Score

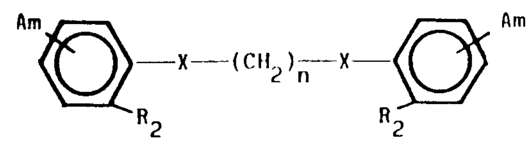

NUMBER OF ANIMALS

| Example No. | | | 0.5 | 1 | 2 | 3 | 4 |
|---|---|---|---|---|---|---|---|
| ---- | | Control | 0 | 1 | 2 | 1 | 4 |
| 12 | C | $R_2$=H, X=O, n=5, Amidine in para position | 1 | 5 | 2 | 0 | 0 |
| 4 | I | $R_2$=H, X=O, n=3, Amidine in para position | 3 | 4 | 1 | 0 | 0 |
| 28 | C | $R_2$=H, X=O, n=5, Amidine in meta position | 3 | 2 | 2 | 0 | 1 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE III

Extent of Disease by Histologic Score

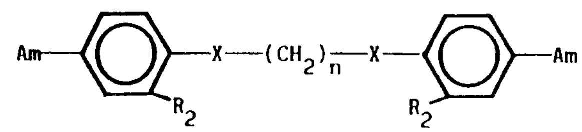

NUMBER OF ANIMALS

| Example No. | | | 0.5 | 1 | 2 | 3 | 4 |
|---|---|---|---|---|---|---|---|
| ---- | | Control | 0 | 0 | 1 | 3 | 4 |
| 12 | C | $R_2$=H, X=O, n=5 | 0 | 3 | 4 | 1 | 0 |
| 20 | C | $R_2$=H, X=O, n=6 | 3 | 3 | 1 | 0 | 0 |
| 15 | II | $R_2=OCH_3$, X=O, n=5 | 2 | 1 | 3 | 0 | 0 |
| 14 | II | $R_2=NH_2$, X=O, n=5 | 2 | 4 | 1 | 1 | 0 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE IV

Extent of Disease by Histologic Score

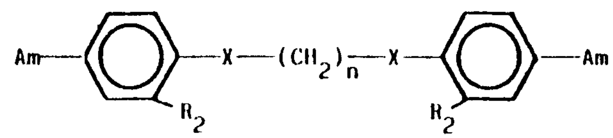

NUMBER OF ANIMALS

| Example No. | | | 0.5 | 1 | 2 | 3 | 4 |
|---|---|---|---|---|---|---|---|
| ---- | | Control | 0 | 0 | 2 | 4 | 2 |
| 12 | C | $R_2$=H, X=O, n=5 | 4 | 4 | 0 | 0 | 0 |
| 9 | I | $R_2$=H, X=O, n=4 | 8 | 0 | 0 | 0 | 0 |
| 19 | II | $R_2$=$NH_2$, X=NH, n=5 | 0 | 2 | 4 | 2 | 0 |
| 13 | II | $R_2$=$NO_2$, X=O, n=5 | 3 | 2 | 3 | 0 | 0 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE V

Extent of Disease by Histologic Score

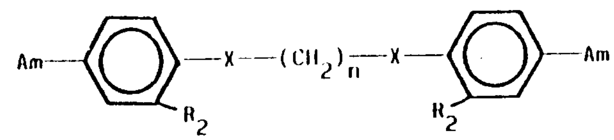

| Example No. | | | NUMBER OF ANIMALS 0.5 | 1 | 2 | 3 | 4 |
|---|---|---|---|---|---|---|---|
| ---- | | Control | 1 | 1 | 3 | 1 | 2 |
| 12 | C | $R_2$=H, X=O, n=5 | 2 | 1 | 3 | 1 | 0 |
| 7 | II | $R_2$=H, X=NH, n=3 | 5 | 1 | 2 | 0 | 0 |
| 8 | II | $R_2$=H, X=NH, n=4 | 3 | 1 | 0 | 0 | 0 |
| 17 | II | $R_2$=H, X=NH, n=5 | 1 | 2 | 3 | 1 | 0 |
| 39 | C | $R_2$=H, X=NH, n=6 | 2 | 1 | 0 | 0 | 0 |
| 21 | C | $R_2$=$NO_2$, X=O, n=6 | 4 | 1 | 1 | 0 | 0 |
| 18 | II | $R_2$=$NO_2$, X=NH, n=5 | 1 | 3 | 0 | 2 | 1 |
| 23 | C | $R_2$=$NH_2$, X=NH, n=6 | 3 | 1 | 2 | 0 | 1 |

Histologic Scoring:
0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE VI

Extent of Disease by Histologic Score

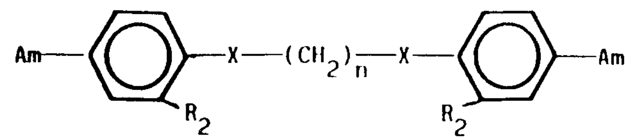

NUMBER OF ANIMALS

| Example No. | | | 0.5 | 1 | 2 | 3 | 4 |
|---|---|---|---|---|---|---|---|
| ---- | | Control | 0 | 0 | 0 | 4 | 4 |
| 12 | C | $R_2$=H, X=O, n=5 | 5 | 3 | 0 | 0 | 0 |
| 3 | II | $R_2$=$NH_2$, X=O, n=2 | 4 | 4 | 0 | 0 | 0 |
| 5 | II | $R_2$=$NH_2$, X=O, n=3 | 2 | 4 | 1 | 0 | 0 |
| 10 | II | $R_2$=$NH_2$, X=O, n=4 | 2 | 5 | 1 | 0 | 0 |
| 14 | II | $R_2$=$NH_2$, X=O, n=5 | 6 | 1 | 0 | 0 | 0 |
| 22 | C | $R_2$=$NH_2$, X=O, n=6 | 0 | 1 | 4 | 3 | 0 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE VII

Extent of Disease by Histologic Score

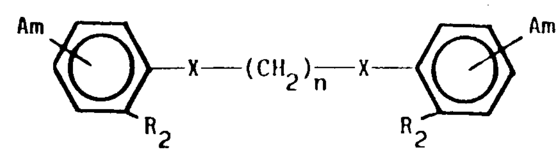

NUMBER OF ANIMALS

| Example No. | | | 0.5 | 1 | 2 | 3 | 4 |
|---|---|---|---|---|---|---|---|
| ---- | | Control | 0 | 0 | 1 | 3 | 4 |
| 12 | C | Pentamidine | 4 | 2 | 2 | 0 | 0 |
| 26 | I | n=3, Am=meta | 2 | 1 | 4 | 1 | 0 |
| 27 | I | n=4, Am=meta | 2 | 1 | 1 | 3 | 0 |
| 28 | C | n=5, Am=meta | 1 | 1 | 4 | 2 | 0 |
| 29 | C | n=6, Am=meta | 1 | 4 | 1 | 2 | 0 |
| ---- | I | n=5, Am=blocked | 5 | 3 | 0 | 0 | 0 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

TABLE VIII

Extent of Disease by Histologic Score

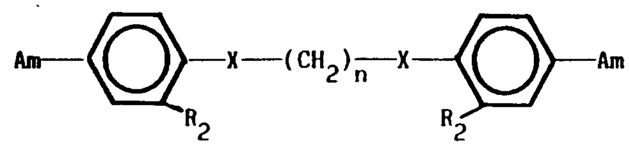

NUMBER OF ANIMALS

| Example No. | | | 0.5 | 1 | 2 | 3 | 4 |
|---|---|---|---|---|---|---|---|
| ---- | | Control | 0 | 0 | 0 | 1 | 7 |
| 12 | C | $R_2$=H, X=O, n=5 | 4 | 2 | 0 | 0 | 0 |
| 6 | II | $R_2$=$OCH_3$, X=O, n=3 | 7 | 1 | 0 | 0 | 0 |
| 11 | II | $R_2$=$OCH_3$, X=O, n=4 | 3 | 4 | 1 | 0 | 0 |
| 15 | II | $R_2$=$OCH_3$, X=O, n=5 | 0 | 3 | 4 | 1 | 0 |
| 24 | C | $R_2$=Cl, X=O, n=4 | 0 | 1 | 2 | 3 | 2 |
| 25 | C | $R_2$=Cl, X=O, n=5 | 0 | 0 | 0 | 4 | 4 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection
C = Comparative, I represents a known compound of Formula I and II represents a novel compound of Formula II

## TABLE IX

Extent of Disease by Histologic Score

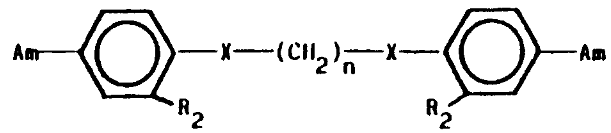

NUMBER OF ANIMALS

| Example No. | | | 0.5 | 1 | 2 | 3 | 4 |
|---|---|---|---|---|---|---|---|
| --- | | Control | 0 | 0 | 0 | 6 | 2 |
| 12 | C | $R_2$=H, X=O, n=5 | 0 | 6 | 1 | 0 | 0 |
| 33 | II | $R_2$=$NH_2$, X=NH, n=2 | 0 | 0 | 4 | 3 | 1 |

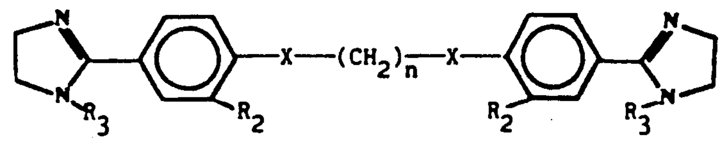

$R_3$ $R_2$ $R_2$ $R_3$

| | | | | | | | |
|---|---|---|---|---|---|---|---|
| 42 | II | $R_3$=$CH_3$, $R_2$=H, X=O, n=5 | 0 | 0 | 2 | 3 | 3 |
| 41 | II | $R_3$=$CH_3$, $R_2$=H, X=O, n=4 | 0 | 0 | 2 | 4 | 2 |
| 44 | II | $R_3$=H, $R_2$=$OCH_3$, X=O, n=3 | 6 | 2 | 0 | 0 | 0 |

Histologic Scoring:
- 0.5 = < 10 cysts found per 2 sections
- 1 = scattered cysts, < 5% of lung involved
- 2 = scattered cysts, 5-10% lung involved or small foci of infection
- 3 = scattered cysts, 10-50% of lung involved with some intense areas of infection
- 4 = > 50% of lung involved with many intense areas of focal infection
- C = Comparative, I represents a known compound of Formula I and II represents a novel compound of Formula II

TABLE X
Extent of Disease by Histologic Score

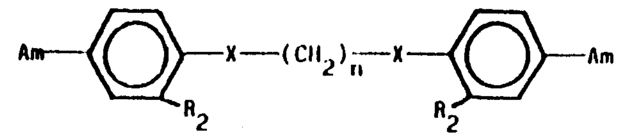

NUMBER OF ANIMALS

| Example No. | | | 0.5 | 1 | 2 | 3 | 4 | |
|---|---|---|---|---|---|---|---|---|
| ---- | | Control | 0 | 0 | 1 | 2 | 5 | ---- |
| 35 | II | $R_2=NO_2$, X=NH, n=3 | 0 | 0 | 0 | 2 | 6 | 5 mg/kg |
| 36 | II | $R_2=NH_2$, X=NH, n=3 | 1 | 4 | 2 | 0 | 0 | 10 mg/kg |
| 37 | II | $R_2=NH_2$, X=NH, n=4 | 0 | 4 | 3 | 1 | 0 | 10 mg/kg |
| 38 | II | $R_2=NO_2$, X=0, n=4 | 0 | 0 | 3 | 1 | 4 | 2.5 mg/kg |
| | | | 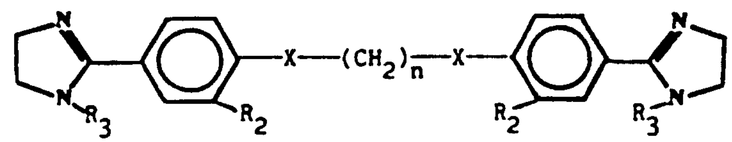 | | | | | |
| | | | $R_3$ | $R_2$ | | $R_2$ | $R_3$ | |
| 40 | II | $R_2$=H, $R_3$=H, X=0, n=4 | 1 | 2 | 3 | 1 | 0 | 10 mg/kg |
| 43 | II | $R_3$=H, $R_2=OCH_3$, X=0, n=5 | 1 | 3 | 1 | 2 | 0 | 5 mg/kg |

Histologic Scoring:
- 0.5 = < 10 cysts found per 2 sections
- 1 = scattered cysts, < 5% of lung involved
- 2 = scattered cysts, 5-10% lung involved or small foci of infection
- 3 = scattered cysts, 10-50% of lung involved with some intense areas of infection
- 4 = > 50% of lung involved with many intense areas of focal infection
- C = Comparative, I represents a known compound of Formula I and II represents a novel compound of Formula II

TABLE XI

Extent of Disease by Histologic Score

| | NUMBER OF ANIMALS | | | | |
|---|---|---|---|---|---|
| | 0.5 | 1 | 2 | 3 | 4 |
| Control | 0 | 0 | 2 | 5 | 5 |
| Pentamidine 10 mg/kg | 3 | 5 | 2 | 1 | 0 |
| Butamidine 10 mg/kg | 10 | 2 | 0 | 0 | 0 |
| Pentamidine 1 mg/kg | 1 | 0 | 3 | 5 | 3 |
| Butamidine 1 mg/kg | 0 | 1 | 3 | 5 | 3 |
| Pentamidine 0.1 mg/kg | 0 | 0 | 0 | 7 | 5 |
| Butamidine 0.1 mg/kg | 0 | 0 | 4 | 5 | 2 |

Histologic Scoring:

0.5 = < 10 cysts found per 2 sections
1 = scattered cysts, < 5% of lung involved
2 = scattered cysts, 5-10% lung involved or small foci of infection
3 = scattered cysts, 10-50% of lung involved with some intense areas of infection
4 = > 50% of lung involved with many intense areas of focal infection

Table XII

EXTENT OF DISEASE BY HISTOLOGIC SCORE (COMBINED)

| Compound Number[b] | Number of Animals per Scoring Group[a] 0.5 | 1 | 2 | 3 | 4 |
|---|---|---|---|---|---|
| Saline | 1 | 2 | 9 | 25 | 35 |
| 1. | Not tested in animals - insoluble | | | | |
| 2. | Not tested in animals - insoluble | | | | |
| 3. | 4 | 4 | 0 | 0 | 0 |
| 4. | 3 | 4 | 1 | 0 | 0 |
| 5. | 2 | 4 | 1 | 0 | 0 |
| 6.[c] | 7 | 1 | 0 | 0 | 0 |
| 7. | 5 | 1 | 2 | 0 | 0 |
| 8. | 3 | 1 | 0 | 0 | 0 |
| 9. | 8 | 0 | 0 | 0 | 0 |
| 10. | 2 | 5 | 1 | 0 | 0 |
| 11.[c] | 3 | 4 | 1 | 0 | 0 |
| 12.(Pentamidine) | 20 | 26 | 12 | 2 | 0 |
| 13.[c] | 0 | 2 | 4 | 2 | 0 |
| 14. | 8 | 5 | 1 | 1 | 0 |
| 15.[d] | 2 | 4 | 7 | 1 | 0 |
| 16. | Not tested in animals - insoluble | | | | |
| 17. | 1 | 2 | 3 | 1 | 0 |
| 18.[c] | 1 | 3 | 0 | 2 | 1 |
| 19. | 3 | 2 | 3 | 0 | 0 |
| 20. | 3 | 3 | 1 | 0 | 0 |
| 21.[e] | 4 | 1 | 1 | 0 | 0 |
| 22.[c] | 0 | 1 | 4 | 3 | 0 |
| 23. | 3 | 1 | 2 | 0 | 1 |
| 24.[d] | 0 | 1 | 2 | 3 | 2 |
| 25.[d] | 0 | 0 | 0 | 4 | 4 |
| 26. | 2 | 1 | 4 | 1 | 0 |
| 27. | 2 | 1 | 1 | 3 | 0 |

TABLE XII (continued)

| Compound Number[b] | Number of Animals per Scoring Group[a] 0.5 | 1 | 2 | 3 | 4 |
|---|---|---|---|---|---|
| 28. | 4 | 3 | 6 | 2 | 1 |
| 29. | 1 | 4 | 1 | 2 | 0 |
| 30. | 5 | 3 | 0 | 0 | 0 |
| 31. | Not tested in animals - synthesis in progress | | | | |
| 32. | Not tested in animals - synthesis in progress | | | | |
| 33. | 0 | 0 | 4 | 3 | 1 |
| 34. | Not tested in animals | | | | |
| 35.[c] | 0 | 0 | 0 | 2 | 6 |
| 36. | 1 | 4 | 2 | 0 | 0 |
| 37. | 0 | 4 | 3 | 1 | 0 |
| 38.[d] | 0 | 0 | 3 | 1 | 4 |
| 39. | Not tested in animals | | | | |
| 40. | 1 | 2 | 3 | 1 | 0 |
| 41.[d] | 0 | 0 | 2 | 4 | 2 |
| 42.[d] | 0 | 0 | 2 | 3 | 3 |
| 43.[c] | 1 | 3 | 1 | 2 | 0 |
| 44.[d] | 6 | 2 | 0 | 0 | 0 |

TABLE XII (continued)

[a] Histologic Scoring:

0.5 = < 10 cysts found per 2 sections

1 = scattered cysts, < 5% of lung involved

2 = scattered cysts, 5-10% of lung involved

3 = scattered cysts, 10-50% of lung involved with some intense focal areas of infection

4 = > 50% of lung involved with many intense areas of focal infection

[b] All compounds were tested at 10 mg/kg unless otherwise indicated

[c] Tested at 5 mg/kg

[d] Tested at 2.5 mg/kg

[e] Tested at 1.25 mg/kg

TABLE XIII

TOXICITY OF AMIDINES

| Compound Number | Effect |
|---|---|
| 1. | Not tested in animals - insoluble |
| 2. | Not tested in animals - insoluble |
| 3. | None |
| 4. | Slight swelling at injection site |
| 5. | None |
| 6. | None - tested at 5 mg/kg only |
| 7. | None |
| 8. | Necrosis at tips of tails, chronic toxicity, 3 deaths by day 7 with 10 mg/kg |
| 9. | None |
| 10. | None |
| 11. | None - tested at 5 mg/kg only |
| 12. | Some hypotension, edematous tails |
| 13. | Acute toxicity, death at 10 mg/kg |
| 14. | None |
| 15. | Severe hypotension, acute toxicity - death at 10 mg/kg |
| 16. | Not tested in animals - insoluble |
| 17. | Chronic toxicity - 2 deaths by day 13 with 10 mg/kg |
| 18. | Acute toxicity - death at 10 mg/kg |
| 19. | Tremors |
| 20. | Some hypotension, edematous tails, strong anticoagulant effect |
| 21. | Acute toxicity - death at 10, 5, and 2.5 mg/kg |
| 22. | Acute toxicity - death at 10 mg/kg |

| Compound Number | Effect |
|---|---|
| 23. | Spasms when injected rapidly |
| 24. | None - tested at 2.5 mg/kg due to solubility |
| 25. | None - tested at 2.5 mg/kg due to solubility |
| 26. | None |
| 27. | None |
| 28. | Slight swelling at injection site |
| 29. | Slightly edematous tails |
| 30. | None |
| 31. | Not tested |
| 32. | Not tested |
| 33. | Some anticoagulant effect |
| 34. | Not tested |
| 35. | Tested at 5 mg/kg due to solubility - no toxicity |
| 36. | None |
| 37. | None |
| 38. | Acute toxicity at 10 mg/kg and 5 mg/kg - tested at 2.5 mg/kg with no toxicity |
| 39. | Not tested in animals |
| 40. | None |
| 41. | Tested at 2.5 mg/kg due to solubility - no toxicity |
| 42. | Acute toxicity at 10 mg/kg and 5 mg/kg - tested at 2.5 mg/kg with no toxicity |
| 43. | Acute toxicity at 10 mg/kg, "quivers" and cardiac arrhythmias - tested at 5 mg/kg with no toxicity |
| 44. | Tested at 2.5 mg/kg due to solubility - no toxicity |

TABLE XIV

GIARDIA LAMBLIA vs. PARA-AMIDINES

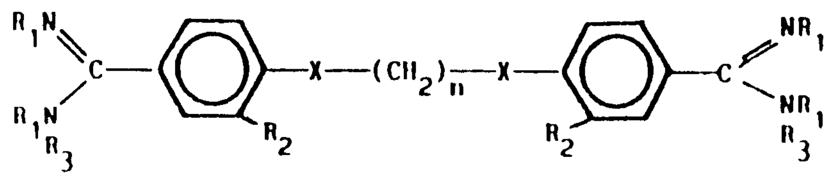

$R_1$, $R_3$ = H

| EXAMPLE NO. | X | n | $R_2$ | $IC_{50}$(uM) |
|---|---|---|---|---|
| 45 | O | 2 | $NH_2$ | 1183.8 |
| 46 | NH | 2 | $NH_2$ | 743.7 |
| 47 | O | 3 | H | 12.3 |
| 48 | O | 3 | $NH_2$ | 15.4 |
| 49 | O | 3 | $OCH_3$ | 3.2 |
| 50 | NH | 3 | H | 68.2 |
| 51 | NH | 3 | $NO_2$ | 84.2 |
| 52 | O | 4 | H | 61.6 |
| 53 | O | 4 | $NH_2$ | 96.8 |
| 54 | O | 4 | $NO_2$ | 53.2 |

**TABLE XIV (continued)**

**GIARDIA LAMBLIA vs. PARA-AMIDINES**

$R_1$, $R_3$ = H

| EXAMPLE NO. | X | n | $R_2$ | $IC_{50}$(uM) |
|---|---|---|---|---|
| 55 | O | 4 | $OCH_3$ | 68.8 |
| 56 | O | 4 | Cl | 27.7 |
| 57 | NH | 4 | H | 50.7 |
| 58 | NH | 4 | $NH_2$ | 107.7 |
| 59 | O | 5 | H | 76.5 |
| 60 | O | 5 | $NH_2$ | 66.3 |
| 61 | O | 5 | $NO_2$ | 26.1 |
| 62 | O | 5 | $OCH_3$ | 24.0 |
| 63 | O | 5 | Cl | 16.3 |
| 64 | NH | 5 | H | 40.6 |
| 65 | NH | 5 | $NH_2$ | 48.6 |
| 66 | NH | 5 | $NO_2$ | 29.9 |
| 67 | O | 6 | H | 34.3 |
| 68 | O | 6 | $NH_2$ | 38.7 |
| 69 | NH | 6 | H | 4.2 |
| 70 | NH | 6 | $NH_2$ | 21.9 |

TABLE XV

GIARDIA LAMBLIA vs. META-AMIDINES

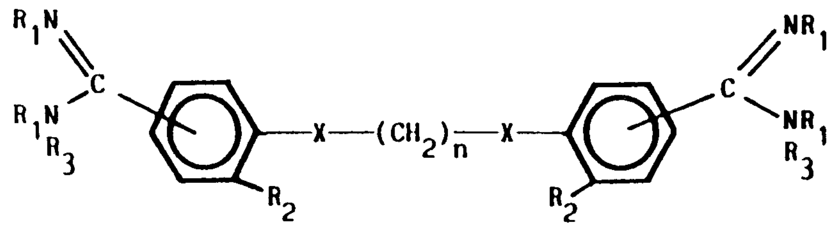

$R_1$, $R_3$ = H

| EXAMPLE NO. | X | n | $R_2$ | $IC_{50}$(uM) |
| --- | --- | --- | --- | --- |
| 71 | 0 | 3 | H | 560.1 |
| 72 | 0 | 4 | H | 105.8 |
| 73 | 0 | 5 | H | 55.8 |
| 74 | 0 | 6 | H | 84.2 |

TABLE XVI

GIARDIA LAMBLIA vs. PARA-IMIDAZOLINES

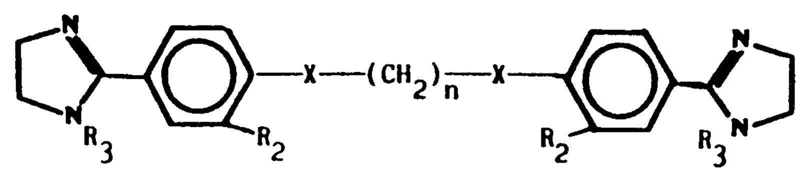

| EXAMPLE NO. | X | n | $R_3$ | $R_2$ | $IC_{50}$(uM) |
|---|---|---|---|---|---|
| 75 | O | 3 | H | $OCH_3$ | 25.4 |
| 76 | O | 4 | H | H | 26.0 |
| 77 | O | 4 | $CH_3$ | H | 346.3 |
| 78 | O | 5 | H | H | 39.2 |
| 79 | O | 5 | $CH_3$ | H | 373.3 |
| 80 | O | 5 | H | $OCH_3$ | 47.1 |

TABLE XVII

COMPOUNDS CURRENTLY USED TO TREAT GIARDIASIS IN THE US AND THEIR $IC_{50}$'S DETERMINED BY $^3H$-THYMIDINE INCORPORATION

| 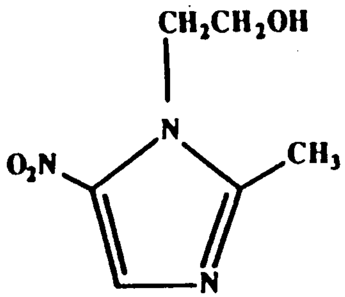 | 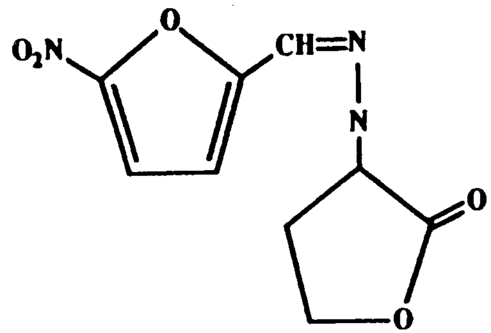 | 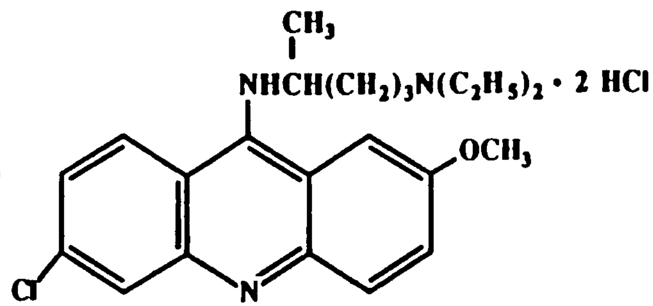 |
|---|---|---|
| Metronidazole | Furazolidone | Quinacrine . HCl |
| $IC_{50}$ = 3.65 μM* | $IC_{50}$ = 1.90 μM* | $IC_{50}$ = 1.41 μM* |
| $IC_{50}$ = 0.91 μM† | $IC_{50}$ = 0.74 μM† | $IC_{50}$ = 1.18 μM† |
| $IC_{50}$ = 2.14 μM§ | $IC_{50}$ = 0.60 μM§ | $IC_{50}$ = 1.09 μM§ |
| $IC_{50}$ - 1.08 μM¶ | $IC_{50}$ = 2.50 μM¶ | $IC_{50}$ = 1.59 μM¶ |
| $IC_{50}$ = 2.21 μM± | $IC_{50}$ = 0.43 μM± | $IC_{50}$ = 4.02 μM± |

* Stock - WB (ATCC 30957)
† Stock - BRIS/83/HEPU/106 (Boreham et al., 1984)
§ Stock - BRIS/82/HEPU/41 (Boreham et al., 1984)
¶ Stock - BRIS/83/HEPU/120 (Boreham et al., 1984)
± Stock - BRI/83/HEPU/136 (Boreham et al., 1984)

TABLE XVIII

LEISHMANIASIS MEXICANA AMAZONENSIS vs. PARA-AMIDINES

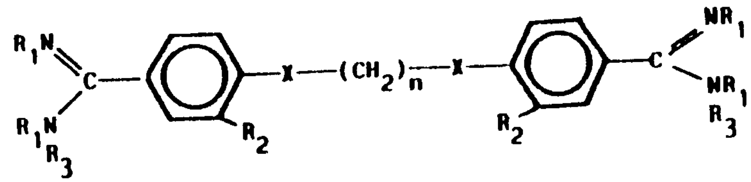

$R_1$, $R_3$ = H

| EXAMPLE NO. | X | n | $R_2$ | (NEW) $IC_{50}$ (uM) | (NEW) Rank | (PREVIOUS) $IC_{50}$ (uM) | (PREVIOUS) Rank |
|---|---|---|---|---|---|---|---|
| 81 | O | 3 | $OCH_3$ | 1.69 | 10 | 1.93 | 12 |
| 82 | NH | 4 | H | 0.58 | 3 | 0.60 | 3 |
| 83 | O | 4 | H | 1.23 | 7 | 1.19 | 9 |
| 84 | O | 5 | H | 0.65 | 4 | 0.75 Ave. | 5 |
| 85 | O | 5 | $NO_2$ | 2.57 | 13 | 1.07 | 8 |
| 86 | O | 5 | $NH_2$ | 2.32 | 12 | 0.95 | 6 |
| 87 | O | 5 | $OCH_3$ | 6.53 | 16 | 2.12 | 13 |
| 88 | NH | 5 | H | 0.35 | 2 | 0.35 | 1 |
| 89 | O | 6 | H | 0.33 | 1 | 0.43 | 2 |
| 90 | O | 5 | Cl | 1.11 | 6 | 1.06 | 7 |

TABLE XIX

LEISHMANIASIS MEXICANA AMAZONENSIS vs. META-AMIDINES

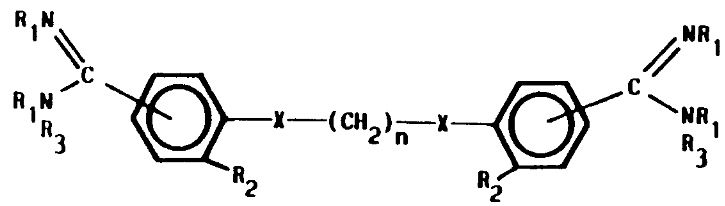

$R_1$, $R_3$ = H

| EXAMPLE NO. | X | n | $R_2$ | (NEW) $IC_{50}$ (uM) | (NEW) Rank (of 18) | (PREVIOUS) $IC_{50}$ (uM) | (PREVIOUS) Rank (of 18) |
|---|---|---|---|---|---|---|---|
| 91 | O | 3 | H | 7.20 | 17 | >4.5 | 17-14 |
| 92 | O | 4 | H | 3.28 | 15 | >4.5 | 17-14 |
| 93 | O | 5 | H | 1.66 | 9 | 1.51 | 11 |
| 94 | O | 6 | H | 2.92 | 14 | 1.35 | 10 |

TABLE XX

LEISHMANIASIS MEXICANA AMAZONENSIS vs. PARA-IMIDAZOLINES

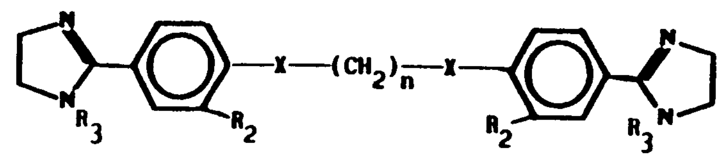

| EXAMPLE NO. | X | n | $R_3$ | $R_2$ | (NEW) $IC_{50}$ (uM) | (NEW) Rank | (PREVIOUS) $IC_{50}$ (uM) | (PREVIOUS) Rank |
|---|---|---|---|---|---|---|---|---|
| 95 | O | 5 | H | H | 1.06 | 5 | 0.67 | 4 |
| 96 | O | 5 | $CH_3$ | H | 12.2 | 18 | >4.5 | 17-14 |
| 97 | O | 3 | H | $OCH_3$ | 1.71 | 11 | >4.5 | 17-14 |
| 98 | O | 3 | H | H | 1.47 | 8 | -- | X |

TABLE XXI

PLASMODIUM FALCIPARUM vs. PARA-AMIDINES

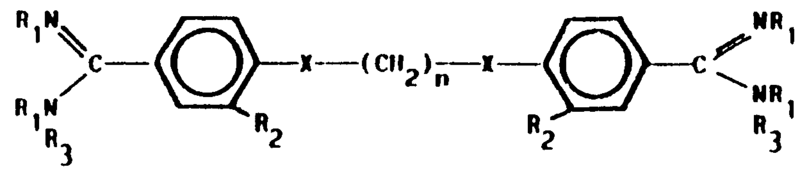

$R_1, R_3 = H$

| EXAMPLE NO. | X | n | $R_2$ | W2 $IC_{50}$(uM) | W2 Rank (of 18) | D6 $IC_{50}$(uM) | D6 Rank (of 18) |
|---|---|---|---|---|---|---|---|
| 99 | O | 3 | $OCH_3$ | 0.065 | 6 | 0.077 | 11 |
| 100 | NH | 4 | H | 0.057 | 3 | 0.025 | 3/4 |
| 101 | O | 4 | H | 0.120 | 13 | 0.057 | 8 |
| 102 | O | 5 | H | 0.083 | 11 | 0.038 | 5 |
| 103 | O | 5 | $NO_2$ | 0.069 | 8 | 0.071 | 10 |
| 104 | O | 5 | $NH_2$ | 0.076 | 9 | 0.046 | 6/7 |
| 105 | O | 5 | $OCH_3$ | 0.066 | 7 | 0.106 | 14 |
| 106 | NH | 5 | H | 0.022 | 1 | <0.020 | 1 |
| 107 | O | 6 | H | 0.345 | 17 | 0.118 | 16 |
| 108 | O | 5 | Cl | 0.165 | 16 | 0.107 | 15 |

TABLE XXII

PLASMODIUM FALCIPARUM vs. META-AMIDINES

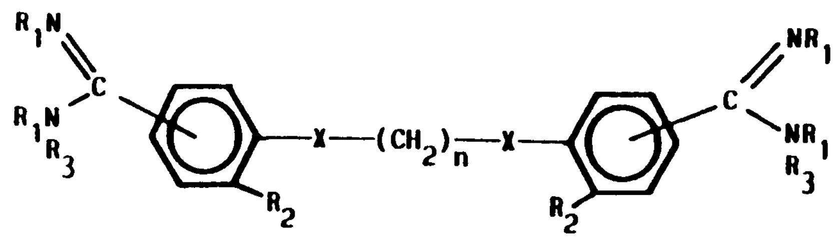

$R_1$, $R_3$ = H

| EXAMPLE NO. | X | n | $R_2$ | W2 $IC_{50}$(uM) | W2 Rank (of 18) | D6 $IC_{50}$(uM) | D6 Rank (of 18) |
|---|---|---|---|---|---|---|---|
| 109 | 0 | 3 | H | 0.156 | 15 | 0.194 | 18 |
| 110 | 0 | 4 | H | 0.043 | 2 | 0.046 | 6/7 |
| 111 | 0 | 5 | H | 0.080 | 10 | 0.069 | 9 |
| 112 | 0 | 6 | H | 0.125 | 14 | 0.132 | 17 |

TABLE XXIII

PLASMODIUM FALCIPARUM vs. PARA-IMIDAZOLINES

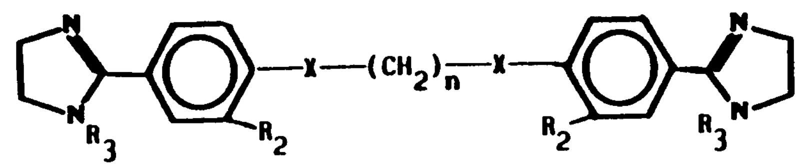

| EXAMPLE NO. | X | n | $R_3$ | $R_2$ | W2 $IC_{50}$(uM) | W2 Rank (of 18) | D6 $IC_{50}$(uM) | D6 Rank (of 18) |
|---|---|---|---|---|---|---|---|---|
| 113 | O | 5 | H | H | 0.104 | 12 | 0.023 | 2 |
| 114 | O | 5 | $CH_3$ | H | 1.817 | 18 | 0.089 | 12 |
| 115 | O | 3 | H | $OCH_3$ | 0.060 | 5/4 | 0.100 | 13 |
| 116 | O | 3 | H | H | 0.060 | 5/4 | 0.025 | 3/4 |

CHART I

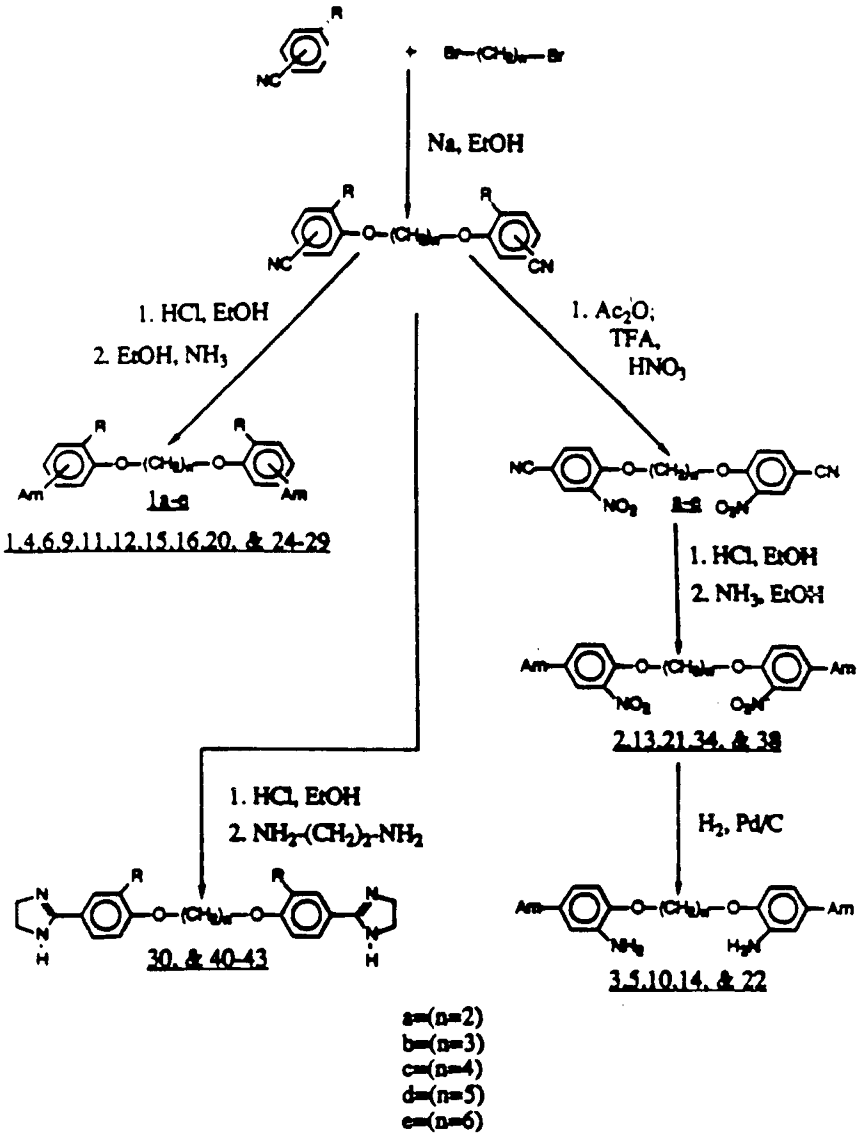
Na, EtOH
1. HCl, EtOH
2. EtOH, NH3
1. Ac2O;
TFA,
HNO3
1. HCl, EtOH
2. NH3, EtOH
H2, Pd/C
1. HCl, EtOH
2. NH2-(CH2)2-NH2
1a-e
1,4,6,9,11,12,15,16,20, & 24-29
2,13,21,34, & 38
30, & 40-43
3,5,10,14, & 22
a=(n=2)
b=(n=3)
c=(n=4)
d=(n=5)
e=(n=6)

CHART II

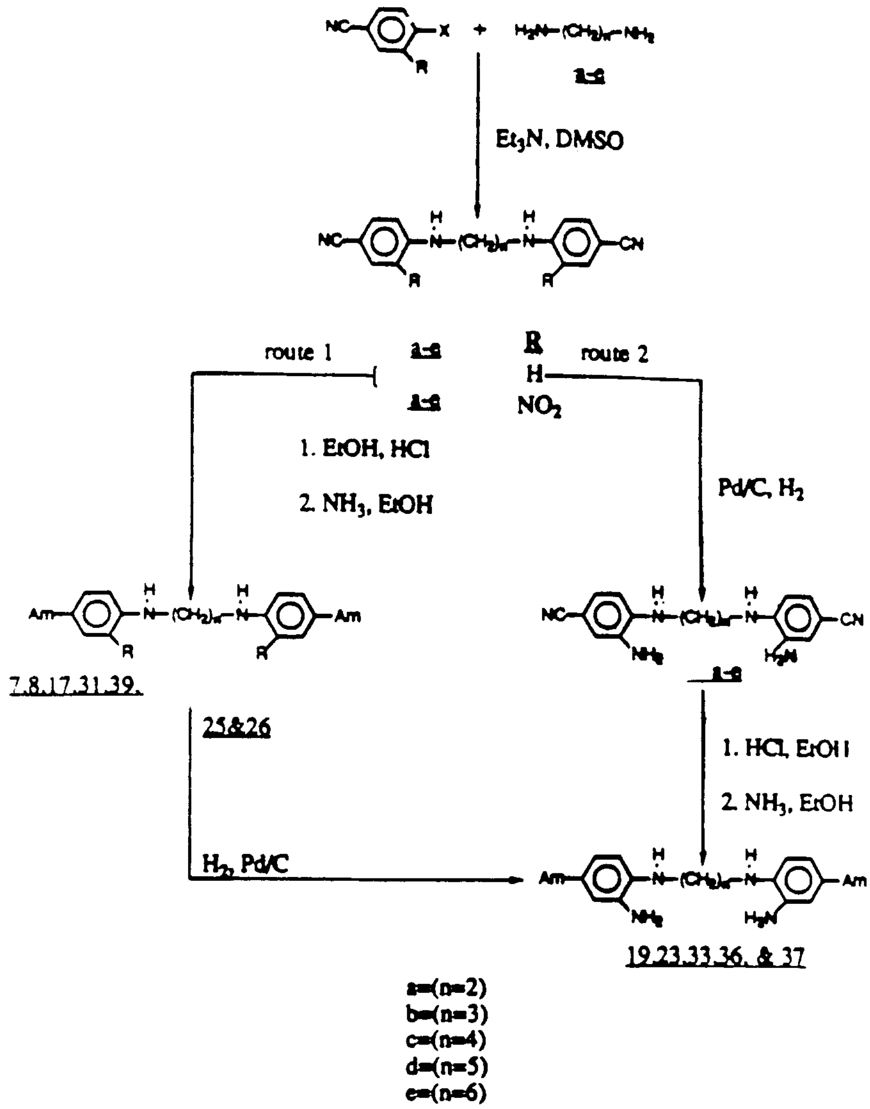
Et$_3$N, DMSO
route 1
route 2
1. EtOH, HCl
2. NH$_3$, EtOH
Pd/C, H$_2$
1. HCl, EtOH
2. NH$_3$, EtOH
H$_2$, Pd/C
7,8,17,31,39,
25&26
19,23,33,36, & 37
a=(n=2)
b=(n=3)
c=(n=4)
d=(n=5)
e=(n=6)

CHART III

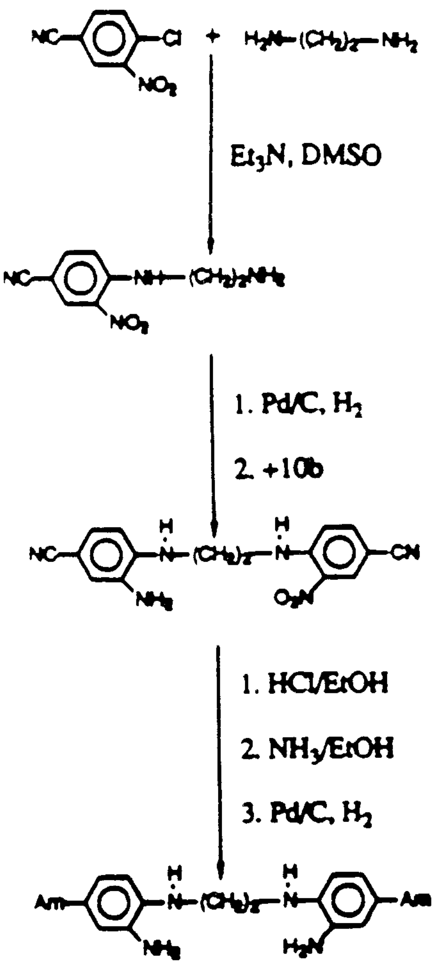


## Claims

**1.** A compound having the structure of Formula II,

II 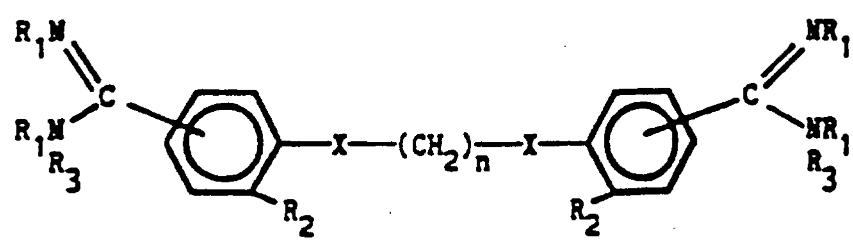

wherein each $R_1$ is H or two $R_1$ groups on the same amidine group together represent $-(CH_2)_m-$, wherein m=2, 3 or 4; $R_2$ is H, $OCH_3$, $NO_2$ or $NH_2$; $R_3$ is H, $CH_3$ or $CH_2CH_3$, n=2, 3, 4 or 5; and X is O, NH or S; with the provisos that when both $R_1$ and $R_2$ are H, then X is NH or S; and when $R_2$ is H and X is O, then two $R_1$ groups together represent $-(CH_2)_m-$ and n=3 or 4; and when X is O or S and n=5, then $R_1$, $R_2$ and $R_3$ are not H at the same time; and when two $R_1$ groups on the same amidine group together represent $-(CH_2)_3-$, X is S and n=5, then $R_2$ and $R_3$ are not H at the same time; and when X is O, two $R_1$ groups on the same amidine group together represent $-(CH_2)_2-$ and n =5, then $R_2$

and $R_3$ are not H at the same time.

**2.** A compound as defined in claim 1 having the following structure,

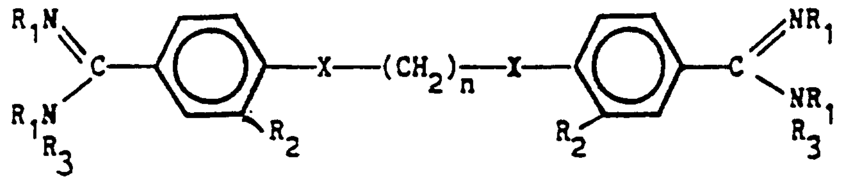

**3.** A compound as defined in Claim 1 having the following structure,

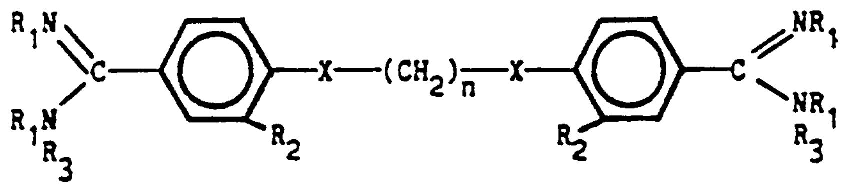

wherein each $R_1$ is H or two $R_1$ groups on the same amidine group together represent $-CH_2CH_2-$; $R_2$ is H, $OCH_3$, $NO_2$ or $NH_2$; and n = 2, 3, 4 or 5, and $R_3$ and X have the meanings of Claim 1.

**4.** A compound of claim 1 having the following structure,

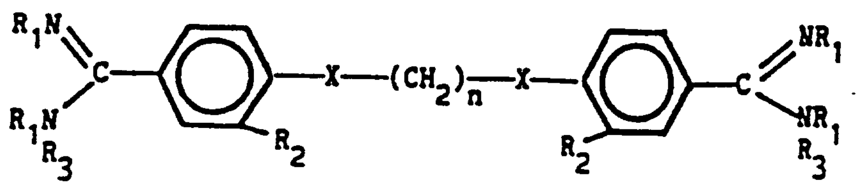

wherein each $R_1$ is H or two $R_1$ groups on the same amidine group together represent $-CH_2CH_2-$; $R_2$ $OCH_3$, $NO_2$ or $NH_2$; X is O or NH; n = 2, 3, 4, or 5, and $R_3$ has the meaning of Claim 1.

**5.** A compound of claim 1 having the following structure,

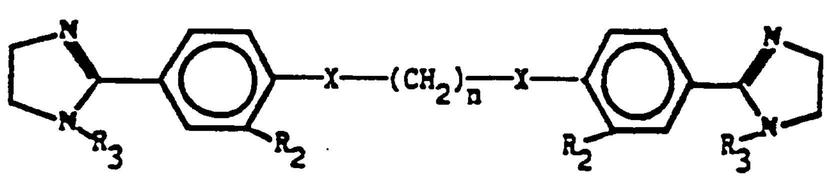

wherein $R_2$ is H, $OCH_3$, $NO_2$ or $NH_2$; X is O or NH; and n = 2, 3, 4 or 5 and $R_3$ has the meaning of Claim 1 provided that when $R_2$ is H, n does not equal 5.

**6.** The compound of claim 1 wherein $R_2 = OCH_3$, $R_3 = H$, X = O, and n = 3.

**7.** A pharmaceutical formulation comprising a therapeutically effective amount of a compound as described in any one of claims 1 to 6, and a pharmaceutically acceptable carrier.

**8.** Use of a compound of formula I for preparing a pharmaceutical formulation for the treatment of Pneumocystis carinii pneumonia

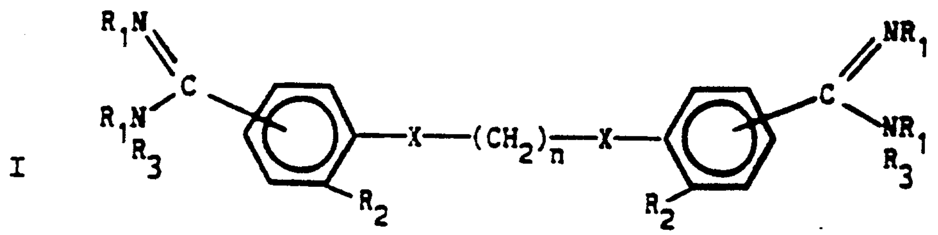

wherein each $R_1$ is H or two $R_1$ groups on the same amidine group together represent $-(CH_2)_m-$, wherein m = 2, 3, or 4; $R_2$ is H, $OCH_3$, $NO_2$ or $NH_2$; $R_3$ is H, $CH_3$, or $CH_2CH_3$; n = 2, 3, 4 or 5; and X is O, NH or S; provided that when both $R_1$ and $R_2$ are H and X = O, then n is not 5.

**9.** Use of a compound of formula I as defined in claim 8 for preparing a pharmaceutical formulation for the treatment of leishmaniasis, or malaria wherein each $R_1$ is H or two $R_1$ groups on the same amidine group together represent $-(CH_2)_m-$, wherein m = 2, 3, or 4; $R_2$ is H, $OCH_3$, $NO_2$, Cl or $NH_2$; $R_3$ is H, $CH_3$, or $CH_2CH_3$; n = 2 - 6; and X is O, NH or S; provided that when both $R_1$ and $R_2$ are H and X = O, then n is not 5, or pharmaceutically acceptable salts thereof.

**10.** Use of a compound of formula I as defined in claim 8 for preparing a pharmaceutical formulation for the treatment of giardiasis wherein each $R_1$ is H or two $R_1$ groups on the same amidine group together represent $-(CH_2)_m-$, wherein m = 2, 3, or 4; $R_2$ is H, $OCH_3$, $NO_2$, Cl or $NH_2$; $R_3$ is H, $CH_3$, or $CH_2CH_3$; n = 2 - 6; and X is O, NH or S or pharmaceutically acceptable salts thereof.

## Patentansprüche

**1.** Verbindung mit der Struktur der Formel II:

II 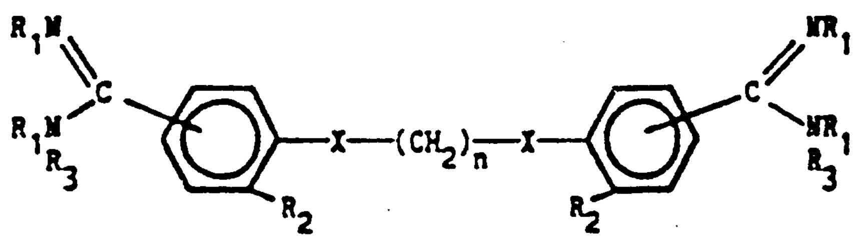

worin jeder der Reste $R_1$ die Bedeutung H hat oder zwei $R_1$-Gruppen an der gleichen Amidingruppe zusammen $-(CH_2)_m-$ darstellen, wobei m = 2, 3 oder 4; $R_2$ die Bedeutung H, $OCH_3$, $NO_2$ oder $NH_2$ hat; $R_3$ die Bedeutung H, $CH_3$ oder $CH_2CH_3$ hat; n = 2, 3, 4 oder 5; und X die Bedeutung O, NH oder S hat; mit der Maßgabe, daß X die Bedeutung NH oder S hat, wenn sowohl $R_1$ als auch $R_2$ die Bedeutung H hat; und daß zwei $R_1$-Gruppen zusammen $-(CH_2)_m-$ darstellen und n = 3 oder 4, wenn $R_2$ die Bedeutung H hat und X die Bedeutung O hat; und daß $R_1$, $R_2$ und $R_3$ nicht gleichzeitig H sind, wenn X die Bedeutung O oder S hat und n = 5; und daß $R_2$ und $R_3$ nicht gleichzeitig H sind, wenn zwei $R_1$-Gruppen an der gleichen Amidingruppe zusammen $-(CH_2)_3-$ darstellen, X die Bedeutung S hat und n = 5 ist; und daß $R_2$ und $R_3$ nicht gleichzeitig H sind, wenn X die Bedeutung O hat, zwei $R_1$-Gruppen an der gleichen Amidingruppe zusammen $-(CH_2)_2-$ darstellen und n = 5.

**2.** Verbindung nach Anspruch 1 mit der folgenden Struktur:

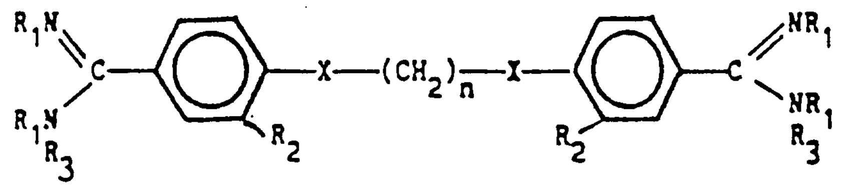

**3.** Verbindung nach Anspruch 1 mit der folgenden Struktur:

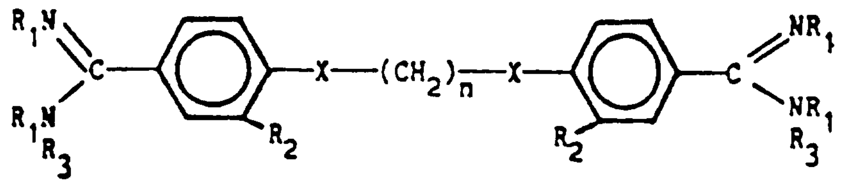

worin jeder der Reste $R_1$ die Bedeutung H hat oder zwei $R_1$-Gruppen an der gleichen Amidingruppe zusammen $-CH_2CH_2-$ darstellen; $R_2$ die Bedeutung H, $OCH_3$, $NO_2$ oder $NH_2$ hat; n = 2, 3, 4 oder 5; und $R_3$ und X die gleiche Bedeutung wie in Anspruch 1 haben.

**4.** Verbindung nach Anspruch 1 mit der folgenden Struktur:

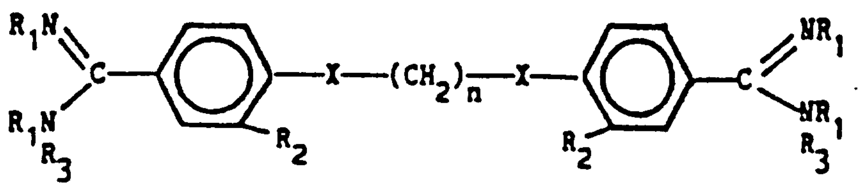

worin jeder der Reste $R_1$ die Bedeutung H hat oder zwei $R_1$-Gruppen an der gleichen Amidingruppe zusammen $-CH_2CH_2-$ darstellen; $R_2$ die Bedeutung $OCH_3$, $NO_2$ oder $NH_2$ hat; X die Bedeutung O oder NH hat; n = 2, 3, 4 oder 5; und $R_3$ die gleiche Bedeutung wie in Anspruch 1 hat.

**5.** Verbindung nach Anspruch 1 mit der folgenden Struktur:

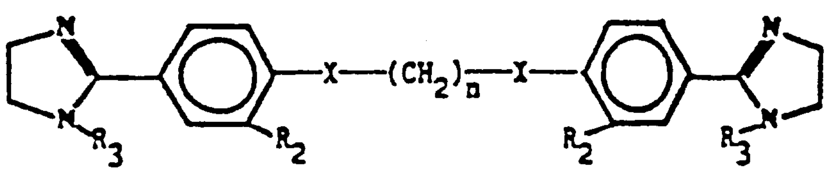

worin $R_2$ die Bedeutung H, $OCH_3$, $NO_2$ oder $NH_2$ hat; X die Bedeutung O oder NH hat; n = 2, 3, 4 oder 5; und $R_3$ die gleiche Bedeutung wie in Anspruch 1 hat, mit der Maßgabe, daß n nicht gleich 5 ist, wenn $R_2$ die Bedeutung H hat.

**6.** Verbindung nach Anspruch 1, worin $R_2 = OCH_3$, $R_3 = H$, $X = O$ und $n = 3$.

**7.** Pharmazeutische Formulierung, umfassend eine therapeutisch wirksame Menge einer Verbindung, wie sie in einem der Ansprüche 1 bis 6 definiert ist, und einen pharmazeutisch verträglichen Träger.

8. Verwendung einer Verbindung der Formel I zur Herstellung einer pharmazeutischen Formulierung zur Behandlung von Pneumocystis carinii-Pneumonie:

I 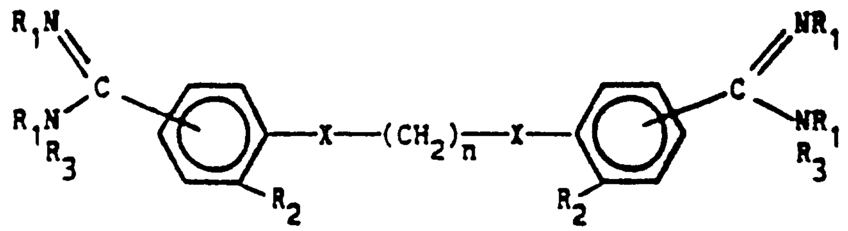

worin jeder der Reste $R_1$ die Bedeutung H hat oder zwei $R_1$-Gruppen an der gleichen Amidingruppe zusammen $-(CH_2)_m-$ darstellen, wobei m = 2, 3 oder 4; $R_2$ die Bedeutung H, $OCH_3$, $NO_2$ oder $NH_2$ hat; $R_3$ die Bedeutung H, $CH_3$ oder $CH_2CH_3$ hat; n = 2, 3, 4 oder 5; und X die Bedeutung O, NH oder S hat; mit der Maßgabe, daß n nicht gleich 5 ist, wenn sowohl $R_1$ als auch $R_2$ die Bedeutung H hat und X = O.

9. Verwendung einer Verbindung der Formel I, wie sie in Anspruch 8 definiert ist, wobei jeder Rest $R_1$ die Bedeutung H hat oder zwei $R_1$-Gruppen an der gleichen Amidingruppe zusammen $-(CH_2)_m-$ darstellen, wobei m = 2, 3 oder 4; $R_2$ die Bedeutung H, $OCH_3$, $NO_2$, Cl oder $NH_2$ hat; $R_3$ die Bedeutung H, $CH_3$ oder $CH_2CH_3$ hat; n = 2-6; und X die Bedeutung O, NH oder S hat; mit der Maßgabe, daß n nicht gleich 5 ist, wenn sowohl $R_1$ als auch $R_2$ die Bedeutung H hat und X = O; oder eines pharmazeutisch verträglichen Salzes davon zur Herstellung einer pharmazeutischen Formulierung zur Behandlung von Leishmaniose oder Malaria.

10. Verwendung einer Verbindung der Formel I, wie sie in Anspruch 8 definiert ist, wobei jeder Rest $R_1$ die Bedeutung H hat oder zwei $R_1$-Gruppen an der gleichen Amidingruppe zusammen $-(CH_2)_m-$ darstellen, wobei m = 2, 3 oder 4; $R_2$ die Bedeutung H, $OCH_3$, $NO_2$, Cl oder $NH_2$ hat; $R_3$ die Bedeutung H, $CH_3$ oder $CH_2CH_3$ hat; n = 2-6; und X die Bedeutung O, NH oder S hat; oder eines pharmazeutisch verträglichen Salzes davon zur Herstellung einer pharmazeutischen Formulierung zur Behandlung von Giardiasis.

**Revendications**

1. Un composé ayant la structure de formule II,

II 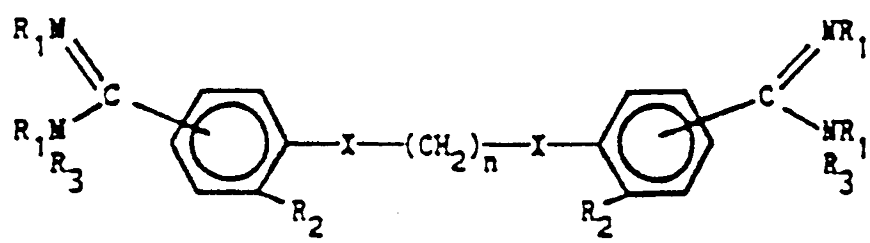

dans laquelle chaque $R_1$ est H ou deux radicaux $R_1$ sur le même groupe amidine représentent ensemble $-(CH_2)_m-$, où m = 2, 3 ou 4; $R_2$ est H, $OCH_3$, $NO_2$ ou $NH_2$; $R_3$ est H, $CH_3$ ou $CH_2CH_3$, n = 2, 3, 4 ou 5; et X est O, NH ou S; à condition que lorsque tant $R_1$ que $R_2$ sont H, alors X est NH ou S; et lorsque $R_2$ est H et X est O, alors deux radicaux $R_1$ représentent ensemble $-(CH_2)_m-$ et n = 3 ou 4; et lorsque X est O ou S et n = 5, alors $R_1$, $R_2$ et $R_3$ ne sont pas H en même temps; et lorsque deux radicaux $R_1$ sur le même groupe amidine représentent ensemble $-(CH_2)_3-$, X est S et n = 5, alors $R_2$ et $R_3$ ne sont pas H en même temps, et lorsque X est O, deux radicaux $R_1$ sur le même groupe amidine représentent ensemble $-(CH_2)_2-$ et n = 5, alors $R_2$ et $R_3$ ne sont pas H en même temps.

2. Un composé selon la revendication 1 répondant à la structure suivante:

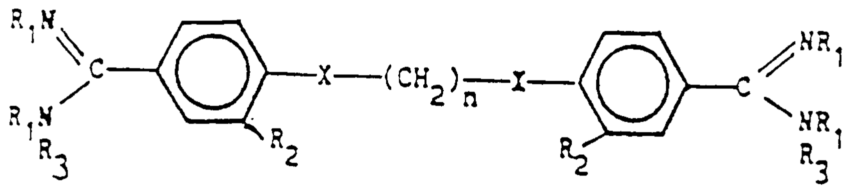

3. Un composé selon la revendication 1, ayant la structure suivante:

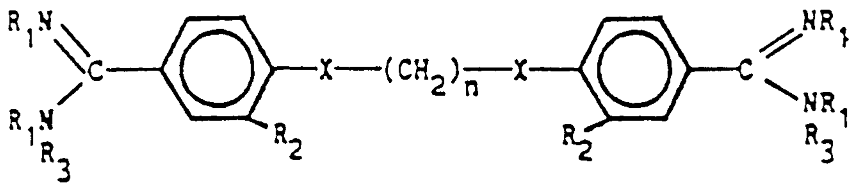

dans laquelle chaque radical $R_1$ est H ou deux radicaux $R_1$ sur le même groupe amidine représentent ensemble $-CH_2CH_2-$; $R_2$ est H, $OCH_3$, $NO_2$ ou $NH_2$ et n = 2, 3, 4 ou 5 et $R_3$ et X ont la même signification que dans la revendication 1.

4. Un composé selon la revendication 1 ayant la structure suivante:

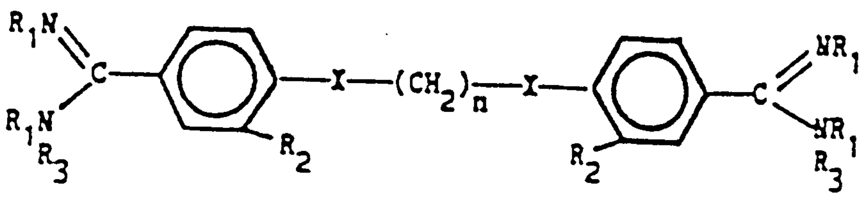

dans laquelle chaque radical $R_1$ est H ou deux radicaux $R_1$ sur le même groupe amidine représentent ensemble $-CH_2CH_2$-$R_2$ est $OCH_3$, $NO_2$ ou $NH_2$; X est O ou NH, n = 2, 3, 4 ou 5, et $R_3$ a la signification mentionnée dans la revendication 1.

5. Un composé selon la revendication 1 ayant la structure suivante:

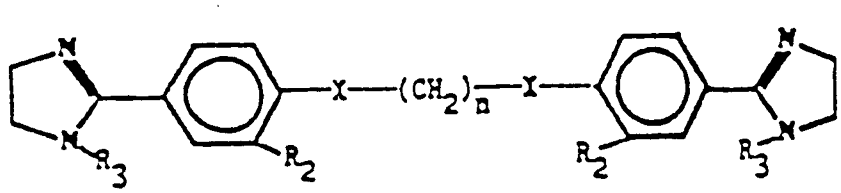

dans laquelle $R_2$ est H, $OCH_3$, $NO_2$ ou $NH_2$, X est O ou NH, et n = 2, 3, 4 ou 5 et $R_3$ a la signification mentionnée dans la revendication 1 à condition que lorsque $R_2$ est H, n ne soit pas égal à 5.

6. Le composé selon la revendication 1, dans lequel $R_2 = OCH_3$, $R_3 = H$, $X = O$, et $n = 3$.

7. Une composition pharmaceutique comprenant une quantité thérapeutiquement efficace d'un composé tel que décrit dans l'une quelconque des revendications 1 à 6, et un véhicule acceptable du point de

vue pharmaceutique.

**8.** utilisation d'un composé de formule I pour préparer une composition pharmaceutique pour le traitement de la pneumonie de pneumocystis carinii:

I 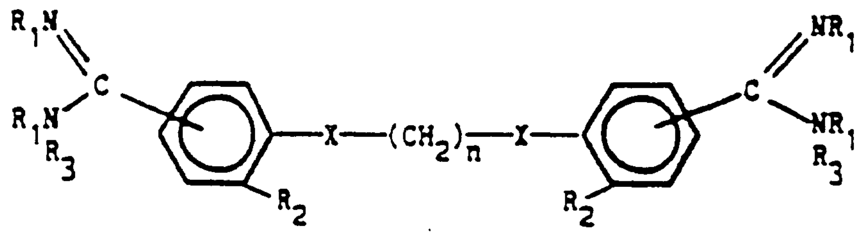

dans laquelle chaque radical $R_1$ est H ou deux radicaux $R_1$ sur le même groupe amidine représentent ensemble $-(CH_2)_m-$, où m = 2, 3 ou 4; $R_2$ est H, $OCH_3$, $NO_2$ ou $NH_2$; $R_3$ est H, $CH_3$ ou $CH_2CH_3$, n = 2, 3, 4 ou 5; et X est O, NH ou S; à condition que lorsque tant $R_1$ que $R_2$ sont H et X=O, n alors n'est pas 5.

**9.** Utilisation d'un composé de formule I selon la revendication 8, pour préparer une composition pharmaceutique pour le traitement de la leishmaniasis ou malaria où chaque radical $R_1$ est H ou deux radicaux $R_1$ sur le même groupe amidine représentent ensemble $-(CH_2)_m-$où m = 2, 3 ou 4; $R_2$ est H, $OCH_3$, $NO_2$, Cl ou $NH_2$; $R_3$ est H, $CH_3$ ou $CH_2CH_3$, n = 2-6; et X est O, NH ou S; à condition que lorsque tant $R_1$ que $R_2$ représentent H et X=O, n alors n'est pas 5, ou de ses sels acceptables du point de vue pharmaceutique.

**10.** Utilisation d'un composé de formule I selon la revendication 8, pour préparer une composition pharmaceutique pour le traitement de la giardiasis où chaque radical $R_1$ est H ou deux radicaux $R_1$ sur le même groupe amidine représentent ensemble $-(CH_2)_m-$ où m = 2, 3 ou 4; $R_2$ est H, $OCH_3$, $NO_2$, Cl ou $NH_2$; $R_3$ est H, $CH_3$ ou $CH_2CH_3$, n = 2-6; et X est O, NH ou S ou de ses sels acceptables du point de vue pharmaceutique.